# EUROPEAN PATENT SPECIFICATION

(11) **EP 1 572 612 B1**
(45) Date of publication and mention of the grant of the patent: **31.10.2007**
(21) Application number: 03780146.1
(22) Date of filing: 10.12.2003
(51) Int. Cl.: C07C 43/225, C07C 49/255, C07C 49/557, C07C 69/66, C07C 205/32, C07C 251/40, C07C 255/32, C07C 271/16, C07D 213/57, C07D 263/32, C07D 307/54, C07D 307/81, C07D 333/24, C07D 213/00

(54) **DIHALO-ALLYLOXY-PHENOL DERIVATIVES HAVING PESTICIDAL ACTIVITY**
DIHALO-ALLYLOXY-PHENOL-DERIVATE MIT PESTIZID-AKTIVITÄT
DERIVES DIHALO-ALLYLOXY-PHENOL A ACTIVITE PESTICIDE

(30) Priority: 11.12.2002 CH 210402
(43) Date of publication of application: 14.09.2005
(73) Proprietor: Syngenta Participations AG, 4058 Basel (CH)
(72) Inventor: ZAMBACH, Werner, Syngenta Crop Protection AG, CH-4058 Basel (CH); RENOLD, Peter, Syngenta Crop Protection AG, CH-4058 Basel (CH); HALL, Roger, Syngenta Crop Protection AG, CH-4058 Basel (CH); TRAH, Stephan, Syngenta Crop Protection AG, CH-4058 Basel (CH)
(74) Representative: Ward, Steven Paul
(86) International application number: PCT/EP2003/014009
(87) International publication number: WO 2004/052816

(56) References cited:
- WO-A-96/33160

## Description

The present invention relates (1) to compounds of formula 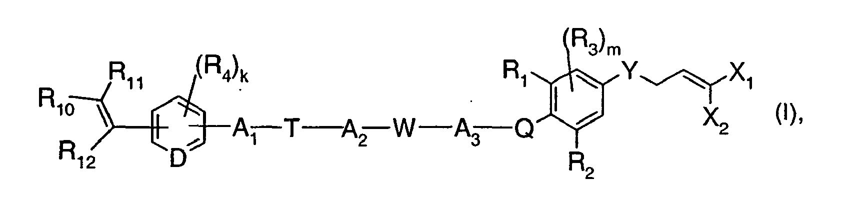 wherein
A₁ and A₂ are each independently of the other a bond or a C₁-C₆alkylene bridge which is unsubstituted or substituted by from one to six identical or different substituents selected from halogen and C₃-C₈cycloalkyl;
A₃ is a C₁-C₆alkylene bridge which is unsubstituted or substituted by from one to six identical or different substituents selected from halogen and C₃-C₈cycloalkyl;
Y is O, NR₇, S, SO or SO₂;
X₁ and X₂ are each independently of the other fluorine, chlorine or bromine;
R_{1'} R₂ and R₃ are each independently of the others H, halogen, OH, SH, CN, nitro, C₁-C₈alkyl, C₁-C₆haloalkyl, C₁-C₆alkylcarbonyl, C₂-C₆alkenyl, C₂-C₆haloalkenyl, C₂-C₆alkynyl, C₁-C₆alkoxy, C₁-C₆haloalkoxy, C₃-C₆alkenyloxy, C₃-C₆haloalkenyloxy, C₃-C₆alkynyloxy, -(S=O)-C₁-C₆alkyl, -(SO)₂-C₁-C₆alkyl or C₁-C₆alkoxycarbonyl; the substituents R₃ being independent of one another when m is 2;
Q is O, NR₅, S, SO or SO₂;
W is O, NR₅, S, SO, SO₂, -C(=O)-O-, -O-C(=O)-, -C(=O)-NR₅- or -NR₅-C(=O)-;
T is a bond, O, NR₅, S, SO, SO₂, -C(=O)-O-, -O-C(=O)-, -C(=O)-NR₅- or -NR₅-C(=O)-;
D is CH or N;
R₄ is H, halogen, OH, SH, CN, nitro, C₁-C₆alkyl, C₁-C₆haloalkyl, C₁-C₆alkylcarbonyl, C₂-C₆alkenyl, C₂-C₆haloalkenyl, C₂-C₆alkynyl, C₁-C₆alkoxy, C₁-C₆haloalkoxy, C₃-C₆-alkenyloxy, C₃-C₆haloalkenyloxy, C₃-C₆alkynyloxy, -(S=O)-C₁-C₆alkyl, -(SO)₂-C₁C₆alkyl, C₁-C₆alkoxycarbonyl or N(R₆)₂ wherein the two substituents R₆ are independent of one another; the substituents R₄ being independent of one another when k is greater than 1;
R₅, R₆ and R₇ are each independently of the others H, C₁-C₆alkyl, C₁-C₃haloalkyl, C₁-C₃haloalkylcarbonyl, C₁-C₆alkoxyalkyl, C₁-C₆alkylcarbonyl, C₁-C₆alkoxycarbonyl, C₃-C₈-cycloalkyl, C₃-C₈cydoalkyl-C₁-C₆alkyl, C₃-C₈cycloalkylcarbonyl;
k is 1, 2 or 3 when D is nitrogen; or is 1, 2, 3 or 4 when D is CH;
m is 1 or 2;
R₁₀ is any radical which comprises from one to three hetero atoms selected from O, N and S; and which may be connected to R₁₂ via a C₁-C₆alkylene bridge;
R₁₁ is H, C₁-C₁₂alkyl, halogen or any radical which comprises from one to three hetero atoms selected from O, N and S; or R₁₁ together with R₁₂ is a bond;
or R₁₀ and R₁₁, together with the carbon atom to which they are bonded, are a five- to seven-membered ring which optionally contains from one to three hetero atoms selected from O, N and S and which is unsubstituted or substituted by from one to three identical or different substituents selected from halogen, OH, =O, SH, =S, =N-OH, =N-O-C₁-C₆alkyl, CN, nitro, C₁-C₆alkyl, C₁-C₆haloalkyl, C₁-C₆alkylcarbonyl, C₂-C₆alkenyl, C₂-C₆haloalkenyl, C₂-C₆alkynyl, C₁-C₆alkoxy and C₁-C₆haloalkoxy;
R₁₂ is H, C₁-C₆alkyl, halo-C₁-C₆alkyl, C₁-C₆alkoxy-C₁-C₆alkyl, C₃-C₆cycloalkyl, phenoxy-C₁-C₆alkyl, CN, -C(=O)C₁-C₁₂alkyl, unsubstituted heterocyclyl, heterocyclyl which is substituted by one to three substituents selected form the group consisting of OH, =O, SH, =S, halogen, CN, nitro, C₁-C₆alkyl, C₁-C₆haloalkyl, C₁-C₆alkylcarbonyl, C₂-C₆alkenyl, C₂-C₆haloaikenyl, C₁-C₆alkoxy and C₁-C₆haloalkoxy; or R₁₂ together with R₁₁ a bond; or is a C₂-C₆alkylene bridge which is connected to R₁₀;
and, where applicable, their possible E/Z isomers, E/Z isomeric mixtures and/or tautomers, in each case in free form or in salt form, to a process for the preparation of those compounds, E/Z isomers and tautomers and to their use in pest control, to pesticidal compositions in which the active ingredient has been selected from those compounds, E/Z isomers and tautomers, and to a process for the preparation of those compositions and to their use, to intermediates and, where applicable, their possible E/Z isomers, E/Z isomeric mixtures and/or tautomers, in free form or in salt form, for the preparation of those compounds, where applicable to tautomers, in free form or in salt form, of those intermediates and to a process for the preparation of those intermediates and their tautomers and to their use.

Certain dihaloallyl derivatives are proposed in the literature as active ingredients in pesticidal compositions. The biological properties of those known compounds are not entirely satisfactory in the field of pest control, however, for which reason there is a need to provide further compounds having pesticidal properties, especially for controlling insects and members of the order Acarina, that problem being solved according to the invention by the provision of the present compounds of formula (I).

The compounds of formula (I) and, where applicable, their tautomers are able to form salts, e.g. acid addition salts. The latter are formed, for example, with strong inorganic acids, such as mineral acids, e.g. sulfuric acid, a phosphoric acid or a hydrohalic acid, with strong organic carboxylic acids, such as unsubstituted or substituted, e.g. halo-substituted, C₁-C₄alkanecarboxylic acids, for example acetic acid, saturated or unsaturated dicarboxylic acids, e.g. oxalic, malonic, maleic, fumaric or phthalic acid, hydroxycarboxylic acids, e.g. ascorbic, lactic, malic, tartaric or citric acid, or benzoic acid, or with organic sulfonic acids, such as unsubstituted or substituted, e.g. halo-substituted, C₁-C₄alkane- or aryl-sulfonic acids, e.g. methane- or p-toluene-sulfonic acid. Furthermore, compounds of formula (I) having at least one acid group are able to form salts with bases. Suitable salts with bases are, for example, metal salts, such as alkali metal or alkaline earth metal salts, e.g. sodium, potassium or magnesium salts, or salts with ammonia or an organic amine, such as morpholine, piperidine, pyrrolidine, a mono-, di- or tri-lower alkylamine, e.g. ethyl-, diethyl-, triethyl- or dimethyl-propyl-amine, or a mono-, di- or tri-hydroxy-lower alkylamine, e.g. mono-, di- or tri-ethanolamine. It may also be possible for corresponding internal salts to be formed. The free form is preferred. Of the salts of compounds of formula (I), preference is given to agrochemically advantageous salts. Hereinabove and hereinbelow any reference to the free compounds of formula (I) or to their salts is to be understood as including, where appropriate, also the corresponding salts or the free compounds of formula (I), respectively. The same applies to tautomers of compounds of formula (I) and their salts.

The general terms used hereinabove and hereinbelow have the meanings given below, unless defined otherwise.

Halogen, as a group *per se* and as a structural element of other groups and compounds, such as haloalkyl, halocycloalkyl, haloalkenyl, haloalkynyl and haloalkoxy, is fluorine, chlorine, bromine or iodine, especially fluorine, chlorine or bromine, more especially fluorine or chlorine, especially chlorine.

Unless defined otherwise, carbon-containing groups and compounds each contain from 1 up to and including 20, preferably from 1 up to and including 18, especially from 1 up to and including 10, more especially from 1 up to and including 6, especially from 1 up to and including 4, especially from 1 up to and including 3, more especially 1 or 2, carbon atoms; methyl is especially preferred.

Alkylene is a straight-chain or branched bridging member and is especially -CH₂-, -CH₂CH₂-, -CH₂-CH₂-CH₂-, -CH₂-CH₂-CH₂-CH₂-, -CH₂-CH₂-CH₂-CH₂-CH₂-, -CH(CH₃)-, -CH₂(CH₃)CH₂-CH₂-, -CH(C₂H₅)-, -C(CH₃)₂-, -CH(CH₃)CH₂-, -CH(CH₃)CH(CH₃)- or -CH₂C(CH₃)₂-CH₂-.

Alkyl, as a group *per se* and as a structural element of other groups and compounds, such as haloalkyl, alkoxy, alkoxyalkyl, haloalkoxy, alkoxycarbonyl, alkylthio, haloalkylthio, alkylsulfonyl and alkylsulfonyloxy, is - in each case giving due consideration to the number of carbon atoms contained in the group or compound in question - either straight-chain, e.g. methyl, ethyl, n-propyl, n-butyl, n-hexyl, n-octyl, n-decyl, n-dodecyl, n-hexadecyl or n-octadecyl, or branched, e.g. isopropyl, isobutyl, sec-butyl, tert-butyl, isopentyl, neopentyl or isohexyl.

Alkenyl and alkynyl - as groups *per se* and as structural elements of other groups and compounds, such as haloalkenyl, haloalkynyl, alkenyloxy, haloalkenyloxy, alkynyloxy or haloalkynyloxy - are straight-chain or branched and each contains two or preferably one unsaturated carbon-carbon bond(s). There may be mentioned by way of example vinyl, prop-2-en-1-yl, 2-methylprop-2-en-1-yl, but-2-en-1-yl, but-3-en-1-yl, prop-2-yn-1-yl, but-2-yn-1-yl and but-3-yn-1-yl.

Cycloalkyl - as a group *per se* and as a structural element of other groups and compounds, such as alkyl - is cyclopropyl, cyclobutyl, cyclopentyl, cyclohexyl, cycloheptyl or cyclooctyl. Cyclopentyl and cyclohexyl, and especially cyclopropyl, are preferred.

Halo-substituted carbon-containing groups and compounds, such as haloalkyl and haloalkoxy, may be partially halogenated or perhalogenated, the halogen substituents in the case of polyhalogenation being the same or different. Examples of haloalkyl - as a group *per se* and as a structural element of other groups and compounds, such as haloalkoxy - are methyl substituted from one to three times by fluorine, chlorine and/or bromine, such as CHF₂, CF₃ or CH₂CI; ethyl substituted from one to five times by fluorine, chlorine and/or bromine, such as CH₂CF₃, CF₂CF₃, CF₂CCI₃, CF₂CHCI₂, CF₂CHF₂, CF₂CFCl₂, CH₂CH₂CI, CF₂CHBr₂, CF₂CHClF, CF₂CHBrF or CCIFCHCIF; propyl or isopropyl each substituted from one to seven times by fluorine, chlorine and/or bromine, such as CH₂CHBrCH₂Br, CF₂CHFCF₃, CH₂CF₂CF₃, CF₂CF₂CF₃, CH(CF₃)₂ or CH₂CH₂CH₂CI; and butyl or an isomer thereof substituted from one to nine times by fluorine, chlorine and/or bromine, such as CF(CF₃)CHFCF₃, CF₂(CF₂)C₂F₃ or CH₂(CF₂)₂CF₃.

Aryl is especially phenyl or naphthyl, preferably phenyl.

Heterocyclyl is to be understood as meaning a five- to seven-membered monocyclic ring which is aromatic or nonaromatic and which contains from one to three hetero atoms selected from the group consisting of N, O and S, especially N and S; or a bicyclic ring system which is aromatic or nonaromatic and which may contain, either in one ring only - as in quinolyl, quinoxalinyl, indolinyl, benzothiophenyl or benzofuranyl, for example - or in both rings - as in pteridinyl or purinyl, for example - one or more hetero atoms selected independently of one another from N, O and S. Preference is given to pyridyl, pyrimidyl, s-triazinyl, 1,2,4-triazinyl, tetrazolyl, thienyl, furanyl, tetrahydrofuranyl, pyranyl, tetrahydropyranyl, pyrrolyl, pyrazolyl, imidazolyl, thiazolyl, triazolyl, oxazolyl, isoxazolyl, thiadiazolyl, oxadiazolyl, benzothienyl, quinolyl, quinoxalinyl, benzofuranyl, benzimidazolyl, benzpyrrolyl, benzthiazolyl, indolyl, coumarinyl or indazolyl, which are preferably bonded *via* a carbon atom; preference is given to thienyl, thiazolyl, benzofuranyl, benzothiazolyl, furanyl tetrahydropyranyl or indolyl; especially pyridyl or thiazolyl.

Preferred embodiments within the scope of the invention are
(2) compounds of formula (I) according to (1) wherein R₁₀ is CN, NO₂, -C(=NOR₁₄)-R₁₃, -C(=O)-R₁₅, -C₁-C₆alkyl-O-R₁₆, -NH-C(=O)-O-R₁₇ or -CH(O-R₁₈)₂;
   R₁₃ is C₁-C₁₂alkyl, C₁-C₆haloalkyl, C₃-C₆cycloalkyl, C₁-C₆alkoxy, C₁-C₃haloalkoxy, C₁-C₆-alkylamino, C₂-C₆alkenyl, C₂-C₆alkynyl, C₂-C₆haloalkenyl, C₂-C₆haloalkynyl; or R₁₃ together with R₁₁ is a C₁-C₆alkylene bridge; or R₁₃ together with R₁₂ a C₃-C₆alkylene bridge; preferably wherein R₁₃ is C₁-C₁₂alkyl, C₁-C₆haloalkyl, C₃-C₆cycloalkyl, C₂-C₆alkenyl, C₂-C₆alkynyl, C₂-C₆haloalkenyl or C₂-C₆haloalkynyl;
   R₁₄ is H, C₁-C₆alkyl, C₃-C₆cycloalkyl-C₁-C₆alkyl, C₃-C₆alkenyl or C₃-C₆alkynyl;
   R₁₅ is H, OH, C₁-C₁₂alkyl, C₁-C₆alkoxy, C₁-C₁₂haloalkyl, C₁-C₆haloalkoxy, C₃-C₆alkenyloxy, C₃-C₆haloalkenyloxy, -N(R₁₈)₂, C₃-C₆cycloalkyl, aryl, aryloxy, benzyloxy or heterocyclyl; or R₁₅ together with R₁₂ is an C₁-C₆alkylene bridge; and
   R₁₆ is H, C₁-C₆alkyl, C₁-C₆haloalkyl, C₃-C₆alkenyl, C₃-C₆haloalkenyl, C₃-C₆alkynyl, C₃-C₆cycloalkyl, C₃-C₆cycloalkyl-C₁-C₆alkyl, C₁-C₆alkoxy-C₁-C₆alkyl, C₁-C₆haloalkoxy-C₁-C₆alkyl, C₁-C₆alkoxy-C₁-C₆alkoxy-C₁-C₆alkyl, C₁-C₆haloalkoxy-C₁-C₆alkoxy-C₁-C₆alkyl, C₃-C₆alkenyloxy-C₁-C₆alkyl, C₃-C₆alkynyloxy-C₁-C₆alkyl, C₃-C₆cydoalkoxy-C₁-C₆alkyl, C₃-C₆cycloalkyl-C₁-C₆alkoxyC₁-C₆alkyl or benzyl;
   R₁₇ is H, C₁-C₆alkyl, C₁-C₆haloalkyl, C₃-C₆alkenyl, C₃-C₆haloalkenyl, C₃-C₆alkynyl, C₃-C₆cycloalkyl, C₃-C₆cycloalkyl-C₁-C₆alkyl, C₁-C₆alkoxy-C₁-C₆alkyl or benzyl;
   the two substituents R₁₈ are each independently of the other C₁-C₁₂alkyl or benzyl or together are a C₂-C₆alkylene bridge;
(3) compounds of formula (I) according to (1) wherein R₁₀ and R₁₁, together with the carbon atom to which they are bonded, are a five- or six-membered ring which optionally contains from one to three hetero atoms selected from O and N and which is unsubstituted or substituted by from one to three identical or different substituents selected from halogen, OH, =O, SH, =S, =N-OH, =N-O-C₁-C₆alkyl, CN, C₁-C₆alkyl, C₁-C₆haloalkyl and C₁-C₆-haloalkoxy;
(4) compounds of formula (I) according to (1) wherein R₁₁ and R₁₂ together are a bond;
(5) compounds of formula (I) according to (1) wherein R₁₀ is CN;
(6) compounds of formula (I) according to (1) wherein R₁₀ is NO₂;
(7) compounds of formula (I) according to (1) wherein R₁₀ is -C(=NOR₁₄)-R₁₃ and R₁₃ is C₁-C₁₂alkyl or C₃-C₆cycloalkyl, especially C₁-C₁₂alkyl;
(8) compounds of formula (I) according to (1) wherein R₁₀ is -C(=O)-R₁₅;
(9) compounds of formula (I) according to (1) wherein R₁₀ is R₁₆-O-C₁-C₆alkyl;
(10) compounds of formula (I) according to (1) wherein R₁₁ is H, C₁-C₁₂alkyl, halogen, CN or -C(=O)-R₁₅ and R₁₅ is as defined under (2);
(11) compounds of formula (I) according to (1) to (10) wherein R₁₂ is H, C₁-C₆alkyl, CN, -C(O)-C₁-C₆alkyl or halo-C₁-C₆alkyl; especially H or C₁-C₆alkyl;
(12) compounds of formula (I) according to (1) to (10) wherein R₁₂ is C₁-C₆alkoxy-C₁-C₆alkyl;
(13) compounds of formula (I) according to (1) to (10) wherein R₁₂ is heteroaryl; preferably pyridyl, isoxazolyl, thienyl, furanyl or benzofuranyl;
(14) compounds of formula (I) according to (1) to (13) wherein X₁ and X₂ are chlorine or bromine, especially chlorine;
(15) compounds of formula (I) according to (1) to (14) wherein D is CH;
(16) compounds of formula (I) according to (1) to (14) wherein D is N;
(17) compounds of formula (I) according to (1) to (16) wherein the group A₁-T-A₂ is a bond;
(18) compounds of formula (I) according to (1) to (18) wherein W is oxygen, -C(=O)O- or -C(=O)NH-; especially O;
(19) compounds of formula (I) according to (1) to (18) wherein A₃ is a straight-chain alkylene bridge; especially ethylene, propylene or butylenes; more especially propylene;
(20) compounds of formula (I) according to (1) to (19) wherein Q is oxygen;
(21) compounds of formula (I) according to (1) to (20) wherein Y is oxygen;
(22) compounds of formula (I) according to (1) to (21) wherein R₁ and R₂ are bromine or chlorine, especially chlorine;
(23) compounds of formula (I) according to (1) to (22) wherein R₃ is hydrogen;
(24) compounds of formula (I) according to (1) to (23) wherein R₄ is hydrogen;
(25) compounds of formula (I) according to (1) to (24) wherein R₅ is H;
(26) compounds of formula (I) according to (1) to (25) wherein R₅ is -C(=O)R₈ or -C(=S)R₈, and R₈ is C₁-C₆alkyl, C₁-C₆aloalkyl, C₂-C₆alkenyl, C₂-C₆haloalkenyl, C₂-C₆alkynyl, C₁-C₆alkoxy, C₁-C₆haloalkoxy, C₃-C₆alkenyloxy, C₃-C₆haloalkenyloxy, C₃-C₆alkynyloxy, C₃-C₆cycloalkyl or phenyl;
(27) compounds of formula (I) according to (1) to (26) wherein R₆ is H or C₁-C₆alkyl;
(28) compounds of formula (I) according to (1) to (26) wherein A₁ is a bond, T is oxygen and A₂ is a C₁-C₆alkylene bridge;
(29) compounds of formula (I) according to (1) to (26) wherein A₁ is a bond, T is -C(=O)O-, wherein the oxygen is bonded to A₂, or -C(=O)NH-, wherein NH is bonded to A₂, and A₂ is a C₁-C₆alkylene bridge.

Special preference is given to the compounds listed in the Tables.

The invention relates also to a process for the preparation of a compound of formula (I), or a salt thereof, wherein
(a) a compound of formula 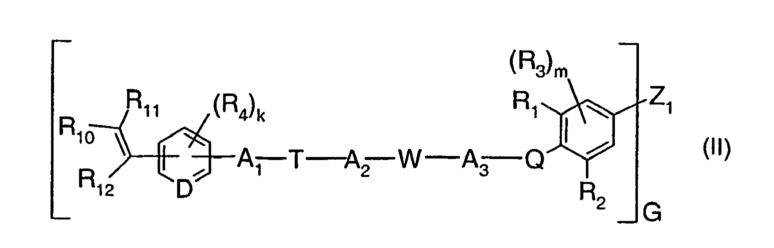 wherein A₁, A₂, A₃, D, W, Q, T, R₁, R₂, R₃, R₄, R₁₀, R₁₁, R₁₂, m and k are as defined for formula (I) under (1), Z₁ is -C(=O)R₂₀ and R₂₀ is H or C₁-C₆alkyl, is converted in the presence of an oxidising agent, especially a peracid, into a compound of formula

   G-Z₂ₐ (llla),

   wherein Z₂ₐ is O-C(=O)-C₁-C₆alkyl and G denotes the part of the formula in brackets designated G in formula (II); either
(b) a compound of formula (llla) above or of formula

   G-Z_{2b} (IIIb),

   wherein G denotes the part of the formula in brackets designated G in formula (II), Z_{2b} is a radical of formula -Y-C(=O)R₂₁, Y is as defined for formula (I) under (1) and R₂₁ is C₁-C₁₂alkyl unsubstituted or substituted by from one to three identical or different halogen substituents, or is phenyl unsubstituted or substituted by from one to three identical or different substituents selected from halogen, CN, nitro, C₁-C₆alkyl, C₁-C₆haloalkyl, C₁-C₆-alkylcarbonyl, C₂-C₆alkenyl, C₂-C₆haloalkenyl, C₂-C₆alkynyl,C₁-C₆alkoxy, C₁-C₆haloalkoxy, C₁-C₆alkoxycarbonyl and C₃-C₆haloalkenyloxy, is converted by hydrolytic cleavage into a compound of formula

   G-Z₃ (IV),

   wherein G denotes the part of the formula in brackets designated G in formula (II), Z₃ is YH and Y is as defined for formula (I) under (1); or
(c) a compound of formula

   G-Z₄ (V),

   wherein Z₄ is Y-CH₂-phenyl, wherein the phenyl radical is unsubstituted or substituted by from one to three identical or different substituents selected from halogen, CN, nitro, C₁-C₆alkyl, C₁-C₆haloalkyl, C₁-C₆alkylcarbonyl, C₂-C₆alkenyl, C₂-C₆haloalkenyl, C₂-C₆alkynyl, C₁-C₆alkoxy, C₁-C₆haloalkoxy, C₁-C₆alkoxycarbonyl and C₃-C₆haloalkenyloxy, G denotes the part of the formula in brackets designated G in formula (II) and Y is as defined for formula (I), is converted by removal of the benzyl group into a compound of formula (IV), as defined above;
(d) the compound of formula (IV) so obtained is reacted in the presence of a base with a compound of formula 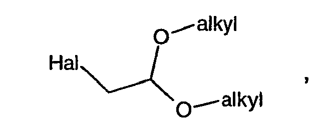 wherein Hal is halogen, preferably bromine or chlorine, and alkyl is C₁-C₆alkyl, or the two alkyl radicals together form a C₂-C₈alkylene bridge, to form a compound of formula

   G-Z₅ (Vl),

   wherein G denotes the part of the formula in brackets designated G in formula (II) and Z₅ is 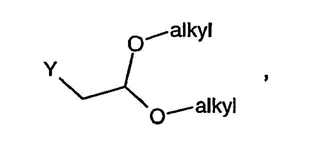 wherein alkyl and Y are as defined above;
(e) the compound of formula (VI) so obtained is converted by deprotection of the acetal function in the presence of an acid into a compound of formula

   G-Z₆ (VII),

   wherein Z₆ is a group -Y-CH₂-C(=O)H, G is as defined above for the compound of formula (II) and Y is as defined for formula (I) under (1), either
(f₁) for the preparation of a compound of formula (I) wherein X₁ and X₂ are chlorine or bromine, a compound of formula (VII) is reacted in the presence of a phosphine with a compound of formula C(X)₄ wherein X is chlorine or bromine; or
(f₂) for the preparation of a compound of formula (I) wherein X₁ and X₂ are chlorine, a compound of formula (Vll) is reacted first with CC1₃-COOH or with chloroform in the presence of a strong base, then with acetic anhydride and subsequently with powdered zinc in acetic acid; or
(f₃) for the preparation of a compound of formula (I) wherein X₁ is fluorine and X₂ is chlorine or bromine, a compound of formula (VII) is reacted in the presence of a phosphine first with a compound of the formula CF₂X₂, of the formula CFX₃, of the formula CF₂XC(=O)ONa or of the formula CFX₂C(=O)ONa; or
(g₁) for the preparation of a compound of formula (I) wherein X₁ and X₂ are chlorine or bromine, a compound of formula (IV) is reacted in the presence of a base with a compound of formula 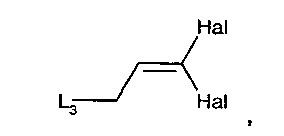 wherein L₃ is a leaving group, preferably chlorine or bromine, and Hal is chlorine or bromine; or
(g₂) for the preparation of a compound of formula (I) wherein X₁ and X₂ are chlorine or bromine, a compound of formula (IVa) or (IVb) is reacted in the presence of a base with a compound of formula 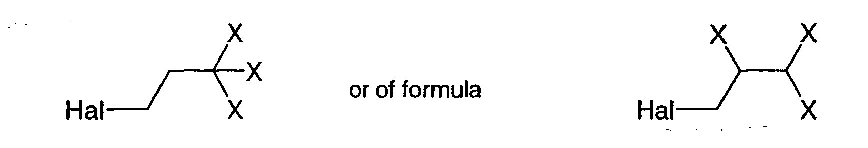 wherein Hal is halogen and X is chlorine or bromine.
   The invention relates also to
(h) a process for the preparation of a compound of formula (I) as defined under (1) and
   wherein Q is O, NR₅ or S and R₅ is as defined for formula (I) under (1), wherein a compound of formula 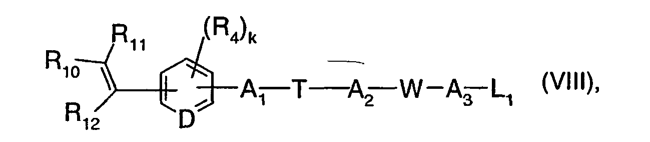 wherein A₁, A₂, A₃, D, W, T, R₄, -R₁₀, R₁₁, R₁₂ and k are as defined for formula (I) under (1) and L₁ is a leaving group, is reacted in the presence of a base with a compound of formula 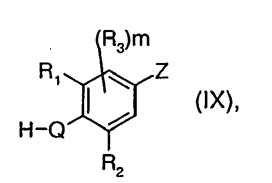 wherein R₁, R₂, R₃ and m are as defined for formula (I) under (1), Q is O, NR₅ or S and Z is one of the radicals Z₁ to Z₆ as defined for the above formulae (II) to (VII), and R₅ is as defined for formula (I) under (1), and the resulting compound of formula 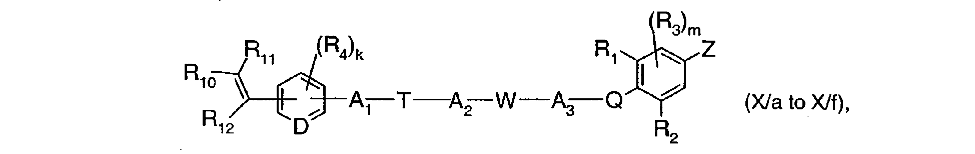 wherein A₁, A₂, A₃, D, W, Q, T, R₁, R₂, R₃, R₄, R₁₀, R₁₁, R₁₂, m and k are as defined for formula (I) under (1) and Z is one of the radicals Z₁ to Z₆ as defined for formulae (II) to (VII) indicated above, is, as necessary, that is to say according to the meaning of the radical Z, reacted further analogously to one or more of process steps (a) to (g).

In the compounds of formulae X/a to X/f, Z in compound X/a has the same meanings as Z₁ in the compound of formula (II), and Z in compound X/b has the same meanings as Z₂ as defined for formula (III), and so on.
The invention relates also to
(i₁) a process for the preparation of a compound of formula (I) as defined above
   wherein W is O, NR₅, S, -O-C(=O)- or -NR₅-C(=O)- and R₅ is as defined for formula (I) under (1), wherein a compound of formula 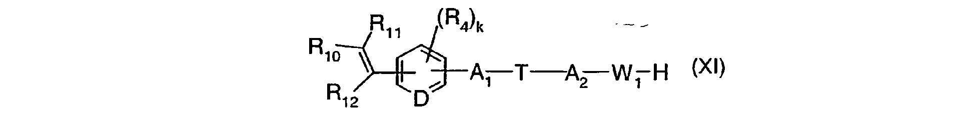 wherein A₁, A₂, A₃, D, T, R₄, R₁₀, R₁₁, R₁₂ and k are as defined for formula (I) under (1), W₁ is O, NR₅ or S and R₅ is as defined for formula (I) under (1), is reacted with a compound of formula 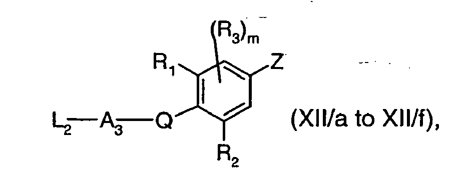 wherein A₃, R₁, R₂, R₃, Q and m are as defined for formula (I) under (1), L₂ is a leaving group or a group Hal-C(=O)- wherein Hal is a halogen atom, preferably chlorine or bromine, and Z is one of the radicals Z₁ to Z₆ as defined in formulae (II) to (VII) indicated above; or
(i₂) for the preparation of a compound of formula (I) as defined above wherein W is O, NR₅, S, -C(=O)-O- or -C(=O)-NR₅- and R₅ is as defined for formula (I) under (1), wherein a compound of formula 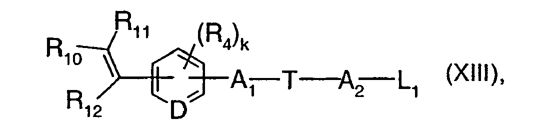 wherein A₁, A₂, D, T, R₄, R₁₀, R₁₁, R₁₂ and k are as defined for formula (I) under (1) and L₁ is a leaving group or a group -C(=O)-Hal wherein Hal is a halogen atom, preferably chlorine or bromine, is reacted with a compound of formula 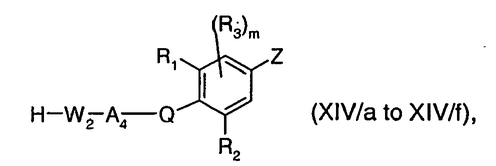 wherein W₂ is O, NR₅ or S and R₁, R₂, R₃ and m are as defined for formula (I) under (1),
   and a resulting compound of formula (Xa) to (Xf) as defined above is, as necessary, that is to say according to the meaning of the radical Z, reacted further analogously to one or more of process steps (a) to (g).

In the compounds of formulae XII/a to Xll/f and XIV/a to XIV/f, the radicals Z are as defined above for the compounds X/a to X/f; that is to say, for example, Z in the compound of formula XII/a has the same meanings as Z₁ in the compound of formula (II), and Z in compound XII/b has the same meanings as Z₂ as defined for formula (III), and so on.

The invention relates also to
(k) a process for the preparation of a compound of formula (I) as defined above under (1), wherein a compound of formula (Vlll) as defined above is reacted in the presence of a base with a compound of formula 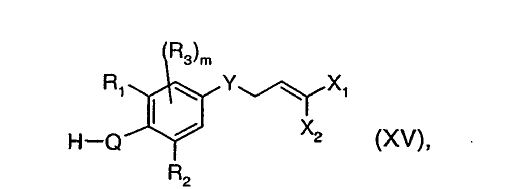 wherein R₁, R₂, R₃, Q, X₁, X₂, Y and m are as defined for formula (I) under (1).
   The invention relates also to
(I) a process for the preparation of a compound of formula (I) as defined above under (1), wherein a compound of formula (XI) as defined above is reacted in a manner analogous to that in process variant (i) with a compound of formula 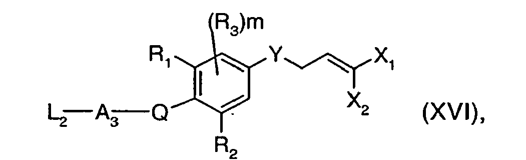 wherein A₃, R₁, R₂, R₃, Q, Y, X₁, X₂ and m are as defined for formula (I) under (1) and L₂ is as defined for formula (XII).

The compounds of formulae (IIIa) and (IIIb) wherein R₁ and R₂ are halogen can be obtained by
(m₁) reacting a compound of formula 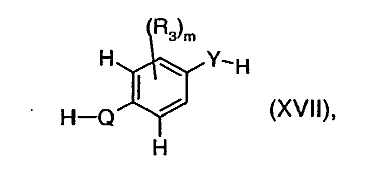 wherein R₃, Q, Y and m are as defined for formula (I) under (1), with a compound of the formula Hal-C(=O)-phenyl wherein Hal is a halogen atom, preferably chlorine or bromine,
(m₂) halogenating the resulting compound of formula 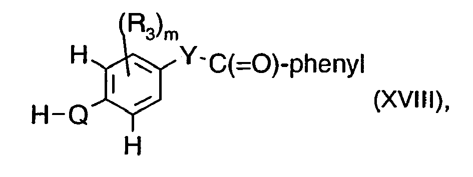 wherein R₃, Q, Y and m are as defined for formula (I) under (1), and further reacting the resulting compound of formula 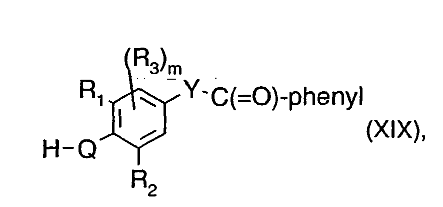 wherein R₃, Q, Y and m are as defined for formula (I) under (1) and R₁ and R₂ are halogen, analogously to Process (k).

The invention relates also to a process
(n) for the preparation of a compound of formula (I) wherein R₁₁ and R₁₂ together are a bond and the remaining radicals are as defined above for formula (I), wherein a compound of formula 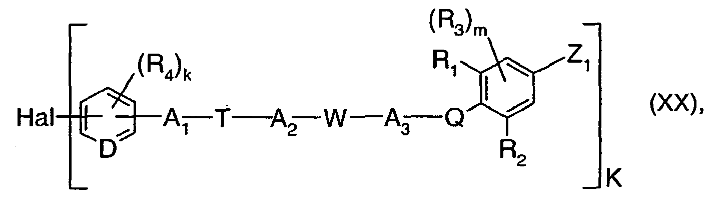 wherein Hal is a halogen atom, preferably bromine or iodine, and A₁, A₂, A₃, D, W, Q, T, R₁, R₂, R₃, R₄, m and k are as defined for formula (I) under (1), is reacted in the presence of a heavy metal catalyst and, where appropriate, of a phosphine with a compound of formula

   R₁₀-C≡CH (XXI),

   wherein R₁₀ is as defined for formula (I), and the resulting compound of formula (I) is, if desired, reacted further to form a different compound of formula (I); by, for example,
(o) for the preparation of a compound of formula (I) wherein R₁₀ is a radical R₁₆-O-C₁-C₆alkyl and R₁₆ is as defined above, alkylating a compound of formula

   K-C=C-C₁-C₆hydroxyalkyl (la)

   obtained according to (n), wherein K corresponds to the expression in brackets in the compound of formula (XX), by means of a compound of formula R₁₆-Hal wherein R₁₆ is as defined above and Hal is a halogen atom; or
(p) for the preparation of a compound of formula (I) wherein R₁₀ is -C(=O)-R₁₃ or -C(=NO-R₁₄)-R₁₃, converting a compound of formula 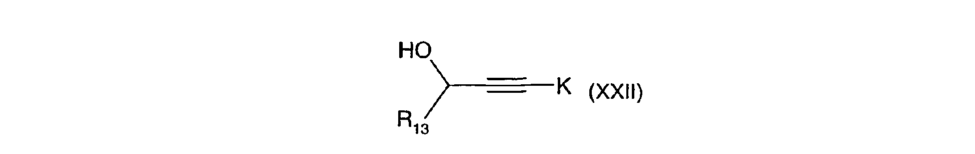 obtainable according to Process (n), wherein K corresponds to the expression in brackets in the compound of formula (XX) and R₁₃ is as defined above for formula (I), by means of an oxidising agent into a compound of formula 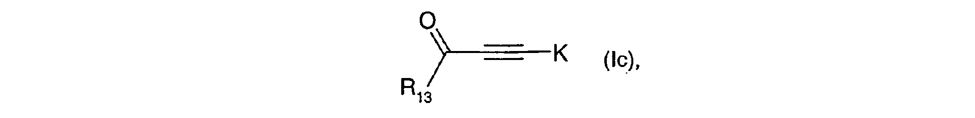 wherein K corresponds to the expression in brackets in the compound of formula (XX) and R₁₃ is as defined above for formula (I), and, if desired, reacting the compound of formula (lc)
(q) with a compound of the formula H₂NOR₁₄ wherein R₁₄ is as defined above for formula (I), or with a salt thereof.

The invention relates also to a process for the preparation of a compound of formula (I) as defined above, wherein
(r) a compound of formula 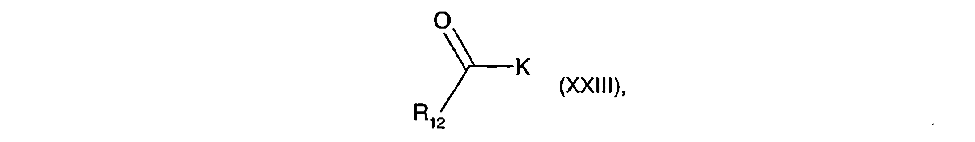 which is known or can be prepared by processes known *per* se and wherein R₁₂ is as defined for formula (I) and K is as defined for formula (XX), is reacted in the presence of a strong base with a compound of formula

   R₁ₒ(R₁₁)P(phenyl)₃ (XXIVa)

   or of formula

   R₁₀(R₁₁)P(=O)(O-alkyl)₂ (XXIVb),

   which are known or can be prepared by processes known *per se* and wherein R₁₀ and R₁₁ are as defined for formula (I) under (1); or
(s) a compound of the above formula (XX) is reacted with a compound of the above formula

   R₁₀(R₁₁)C=CH₂ (XXV),

   which is known or can be prepared by processes known *per se* and wherein R₁₀ and R₁₁ are as defined for formula (I) under (1); or
(t) for the preparation of a compound of formula (I), wherein a compound of formula (XXIII) defined above is reacted with a compound of formula

   R₁₀CH₂R₁₁ (XXVI),

   which is known or can be prepared by processes known *per se* and wherein R₁₀ and R₁₁ are as defined for formula (I) under (1).

It will be understood that Processes (n) to (t) according to the invention may also be carried out on any desired precursor, and those precursors can then be processed according to Processes (a) to (m) to form the compounds of formula (I). The invention relates also to corresponding intermediates. Such intermediates, where novel, are the compounds of formulae (II) to (XXVI), in free form or in salt form, and compounds of formulae 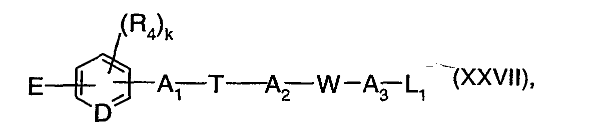 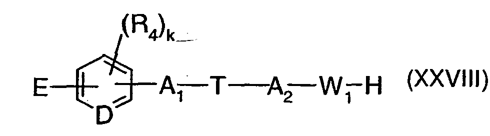 and 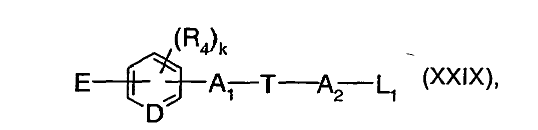 wherein A₁, A₂, A₃, L₁, T, W₁, R₄, d and k are as defined above and E is halogen or a group -CHO, -C(=O)-alkyl or -C(=O)-O-alkyl. It will be understood that such intermediates may have to be protected by protecting groups for further processing, for example by protecting a keto function in ketal form.

The same preferred meanings as defined for the compounds of formula (I) under (2) to (29) apply to the intermediates of formulae (II) to (XXIX).

The reactions described hereinabove and hereinbelow are carried out in a manner known *per se,* for example in the absence or, where appropriate, in the presence of a suitable solvent or diluent or of a mixture thereof, the reactions being carried out, as required, with cooling, at room temperature or with heating, for example in a temperature range of approximately from -80°C to the boiling temperature of the reaction mixture, preferably from approximately -20°C to approximately +150°C, and, if necessary, in a closed vessel, under pressure, under an inert gas atmosphere and/or under anhydrous conditions. Especially advantageous reaction conditions can be found in the Examples.

A leaving group, for example the leaving groups L₁ and L₂ defined above, or a counterion is to be understood hereinabove and hereinbelow as being any removable group that customarily comes into consideration for chemical reactions, such as are known to the person skilled in the art; especially OH, halogens, such as fluorine, chlorine, bromine, iodine, -O-Si(C₁-C₈alkyl)₃, -O-aryl, -S-(C₁-C₈alkyl), -S-aryl, -O-S(=O)₂U, -S(=O)U or -S(=O)₂U, wherein U is unsubstituted or substituted C₁-C₈alkyl, C₂-C₈alkenyl, C₂-C₈alkynyl, unsubstituted or substituted aryl or unsubstituted or substituted benzyl. Especially preferred as leaving group are chlorine, bromine, mesylate, triflate, tosylate, especially chlorine; and chloride or bromide, especially chloride.
Process (a): The reaction is carried out in acetic acid or a halogenated hydrocarbon, such as dichloromethane, at temperatures of from -20°C to 100°C, preferably at from 20°C to 50°C. As oxidising agents there are used, for example, hydrogen peroxide, a peracid, such as peracetic acid, trifluoroperacetic acid or 3-chloroperbenzoic acid, or a mixture thereof, such as sodium perborate in acetic acid.
Process (b): The reaction is preferably carried out in an alcohol, such as methanol, ethanol or an alcohol/water mixture, in the presence of an inorganic base, such as NaOH or KOH, and at temperatures of from 0°C to 150°C, preferably from 20°C to 80°C. Alternatively aminolysis with a primary amine, such as n-butylamine, can be carried out in a hydrocarbon, such as toluene or benzene, at temperatures of from 0°C to 150°C, preferably at from 20°C to 80°C.
Process (c): Depending upon the nature of the benzyl substituent to be removed, the reaction can be carried out, for example, under a hydrogen atmosphere, at a pressure of from 1 to 150 bar, especially at from 1 to 20 bar, and with the addition of a catalyst, such as palladium/carbon, in an alcohol or ether. The preferred reaction temperature is from 0°C to 120°C, especially from 20°C to 80°C.
Processes (d) and (g): The reactions are preferably carried out in the presence of a base, such as potassium or sodium carbonate, in acetone or dimethylformamide, at temperatures of from 0°C to 150°C, preferably from 20°C to 80°C. If necessary, catalytic amounts of potassium iodide or sodium iodide, or phase transfer catalysts, such as crown ethers or quaternary ammonium salts, are added.
Process (e): The reaction is preferably carried out in acetone, dichloromethane, acetic acid, or especially in water, optionally with the addition of a mineral acid, at temperatures of from 0°C to 120°C, preferably at from 20°C to 50°C. For complete cleavage of the acetal it is preferable to add a strong mineral acid, for example hydrochloric acid, sulfuric acid or 4-toluenesulfonic acid.
Process (f): For the preparation of the difluoro-, dichloro-, dibromo-, chlorofluoro- and bromofluoro-vinyl compounds, reaction with CCl₄, CBr₄, CF₂X₂, CFX₃, CF₂XC(=O)ONa or CFX₂C(=O)ONa wherein X is bromine or chlorine is carried out in the presence of a trialkyl- or triaryl-phosphine, optionally with the addition of powdered zinc. The reaction is carried out in an inert solvent, for example benzene or toluene, or an ether, such as diethyl ether, diisopropyl ether, dioxane or tetrahydrofuran, at temperatures of from 0°C to 150°C, preferably at from 20°C to 80°C.
   For the preparation of the dichlorovinyl compounds it is also possible for the process to be carried out in dimethylformamide, benzene, toluene, or in an ether, at temperatures of from 0°C to 120°C, preferably at from 20°C to 80°C, and in the presence of trichloroacetic acid/sodium trichloroacetate, then by addition of acetic anhydride, optionally with the addition of base, for example triethylamine, and finally by addition of zinc and acetic acid.
Processes (h) and (k): The reactions are preferably carried out in an ether, dimethylformamide, dimethylacetamide or N-methylpyrrolidone, at temperatures of from 0°C to 150°C, preferably at from 20°C to 80°C, with the addition of a-base, such as potassium or sodium carbonate. Alternatively a coupling reagent, for example azodicarboxylic acid diethyl or diisopropyl ester and triphenylphosphine, can be used.

### Processes (i) and (I):

Where L₂ is a group Hal-C(=O)-, the process can be carried out in an inert solvent, such as in an ether or in toluene, at from 0°C to 80°C, and in the presence of a suitable base, for example a trialkylamine.

In the other cases the reaction is carried out in an ether, in an amide, such as dimethylformamide or N-methylpyrrolidone, and at from 0°C to 150°C. Sodium hydride, for example, can be used as base.

### Process (m₁):

The reaction is preferably carried out in a solvent, such as dioxane, dichloromethane, acetonitrile or toluene, at from 0 to 100°C, and in the presence of a base.

### Process (m₂):

The reaction is preferably carried out in water or a chlorinated hydrocarbon and using a halogenating agent, such as chlorine, bromine, NaOCI or tert-butyl hypochlorite.

### Process (n):

The reaction is preferably carried out in dimethylformamide, dimethylacetamide, N-methylpyrrolidone, dioxane, tetrahydrofuran, dimethoxyethane or acetonitrile at from 0 to 200°C, preferably at from 50 to 120°C.

Suitable catalysts are, for example, Pd(OAc)₂, PdCl₂, PdCl₂(PPh₃) and Pd(PPh₃)₄; a phosphine, for example a triaryl- or trialkyl-phosphine, may be added where appropriate.

Especially preferred reaction conditions can be found in Example P2.1.

### Process (o):

The reaction is preferably carried out in an amide, such as dimethylformamide, dimethylacetamide or N-methylpyrrolidone, in dioxane, dimethoxyethane, acetonitrile, tetrahydrofuran, using an alkyl halide at from 0°C to 120°C, preferably at from 20° to 80°C.

Suitable bases are, for example, potassium carbonate, sodium carbonate and sodium hydride. A phase transfer catalyst, for example a tetraalkylammonium salt, is added where appropriate.

The process conditions can be found *inter alia* in Example P.3.

### Process (p):

There is used as oxidising agent, for example, sodium bichromate or chromium trioxide in aqueous sulfuric acid, with a solvent such as acetone; at a working temperature of from 0°C to about 60°C; or with pyridinium dichromate in dichloromethane at -40°C. In an alternative procedure it is possible to use a mixture of dimethyl sulfoxide and oxalyl chloride, in dichloromethane as solvent and in the presence of a base, for example triethylamine, preferably at a working temperature of from -70°C to +40°C.

Especially preferred reaction conditions can be found in Example P.4.

### Process (g):

The process is preferably carried out in an alcohol, for example methanol, ethanol or isopropanol, in an amide, such as dimethylformamide, dimethylacetamide, N-methylpyrrolidone, in an ether, such as dioxane or dimethoxyethane, in pyridine or in acetonitrile; at from 0 to 120°C, preferably at from 20 to 80°C.

A base, for example potassium carbonate, sodium carbonate, sodium acetate, potassium acetate, triethylamine, ethyldiisopropylamine or sodium hydride, is preferably used.

Compounds of formula (I) wherein R₁₄ is hydrogen can be reacted further by alkylation with a compound R₁₄-Hal, wherein R₁₄ is as defined above for formula (I), except for the meaning H, and Hal is a halogen atom. That reaction is preferably carried out in dimethylformamide, dimethylacetamide, N-methylpyrrolidone, dioxane, dimethoxyethane, acetonitrile, tetrahydrofuran with an alkyl halide at from 0 to 120°C, preferably at from 20 to 80°C.

A phase transfer catalyst, for example a tetraalkylammonium salt, is used where appropriate.

Especially preferred process conditions can be found in Example P.5.

### Process (r):

The reaction is preferably carried out in toluene, tetrahydrofuran, dioxane, dimethoxyethane, dimethylformamide or dichloromethane at from -60 to 150°C, preferably at from 20°C to 80°C, and in the presence of a base.

There is used as the base, for example, potassium tert-butoxide, sodium tert-butoxide, butyllithium, sodium hydride, lithium diisopropylamine, sodium methoxide or an organic base, such as triethylamine, ethyldiisopropylamine, 1,8-diazabicyclo[5.4.0]undec-7-ene, in the presence of lithium chloride or lithium bromide.

Especially preferred reaction conditions can be found in Example P.1.

### Process (s):

The reaction is preferably carried out using compounds of formula (XX) wherein Hal is chlorine, bromine or iodine. Suitable solvents are amides, such as dimethylformamide, dimethylacetamide and N-methylpyrrolidone, ethers, for example dioxane or dimethoxyethane, or acetonitrile. The reaction is carried out at from 0°C to 200°C, preferably at from 50°C to 120°C.

The reaction is carried out with the use of a palladium catalyst, such as Pd(OAc)₂, PdCl₂ or PdCl₂(PPh₃), where appropriate in the presence of a triaryl- or trialkyl-phosphine, and of a base, such as triethylamine, lithium diisopropylamide, sodium carbonate, potassium carbonate, caesium carbonate or sodium hydride.

### Process (t):

The reaction can be carried out on the one hand in an alcohol, for example methanol or ethanol, in dimethylformamide, N-methylpyrrolidone or tetrahydrofuran as solvent, in the presence of a base, for example diethylamine, diisopropylamine, piperidine, KOH, NaOH or sodium methoxide, at a temperature of from -70°C to the boiling temperature of the solvent; on the other hand it can also be carried out, for example, in acetic acid at from 20°C to 120°C in the presence of ammonium acetate.

Process conditions can be found *inter alia* in Example P.6.

The invention relates especially to the preparation processes described in Examples P1 to P11.

Compounds of formula (I) obtainable in accordance with the process or by other means can be converted into other compounds of formula (I) in a manner known *per* se by replacement of one or more substituents in the starting compound of formula (I) in customary manner by another (other) substituent(s) according to the invention.

In the case of such replacement, depending upon the choice of reaction conditions and starting materials suitable therefor, it is possible for only one substituent to be replaced by another substituent according to the invention in a reaction step or for a plurality of substituents to be replaced by other substituents according to the invention in the same reaction step.

Salts of compounds of formula (I) can be prepared in a manner known *per* se. For example, salts of compounds of formula (I) with bases are obtained by treatment of the free compounds with a suitable base or with a suitable ion exchange reagent.

Salts of compounds of formula (I) can be converted into the free compounds of formula (I) in customary manner, for example by treatment with a suitable acid or with a suitable ion exchange reagent.

Salts of compounds of formula (I) can be converted in a manner known *per* se into other salts of a compound of formula (I).

The compounds of formula (I), in free form or in salt form, may be in the form of one of the possible isomers or in the form of a mixture thereof, for example, depending upon the number of asymmetric carbon atoms occurring in the molecule and their absolute and relative configuration, and/or depending upon the configuration of non-aromatic double bonds occurring in the molecule, in the form of pure isomers, such as antipodes and/or diastereoisomers, or in the form of mixtures of isomers, such as mixtures of enantiomers, for example racemates, mixtures of diastereoisomers or mixtures of racemates. The invention relates both to the pure isomers and to all possible mixtures of isomers and is to be interpreted as such hereinbefore and hereinafter, even if stereochemical details are not mentioned specifically in every case.

Mixtures of diastereoisomers, mixtures of racemates and mixtures of double bond isomers of compounds of formula (I), in free form or in salt form, which may be obtained in accordance with the process - depending upon the starting materials and procedures chosen - or by some other method, can be separated into the pure diastereoisomers or racemates in known manner on the basis of the physico-chemical differences between the constituents, for example by means of fractional crystallisation, distillation and/or chromatography.

Mixtures of enantiomers, such as racemates, that are obtainable in a corresponding manner can be resolved into the enantiomers by known methods, for example by recrystallisation from an optically active solvent, by chromatography on chiral adsorbents, for example high pressure liquid chromatography (HPLC) on acetylcellulose, with the aid of suitable microorganisms, by cleavage with specific, immobilised enzymes, *via* the formation of inclusion compounds, for example using chiral crown ethers, only one enantiomer being complexed, or by conversion into diastereoisomeric salts and separation of the mixture of diastereoisomers so obtained, for example on the basis of their different solubilities by fractional crystallisation, into the diastereoisomers, from which the desired enantiomer can be freed by the action of suitable agents.

Apart from by separation of corresponding mixtures of isomers, pure diastereoisomers or enantiomers can be obtained according to the invention also by generally known methods of diastereoselective or enantioselective synthesis, for example by carrying out the process according to the invention using starting materials having correspondingly suitable stereochemistry.

In each case it is advantageous to isolate or synthesise the biologically more active isomer, e.g. enantiomer or diastereoisomer, or isomeric mixture, e.g. enantiomeric mixture or diastereoisomeric mixture, where the individual components have different biological activity.

The compounds of formula (I), in free form or in salt form, can also be obtained in the form of their hydrates and/or may include other solvents, for example solvents which may have been used for the crystallisation of compounds in solid form.

The invention relates to all those embodiments of the process according to which a compound obtainable as starting material or intermediate at any stage of the process is used as starting material and some or all of the remaining steps are carried out or a starting material is used in the form of a derivative or salt and/or its racemates or antipodes or, especially, is formed under the reaction conditions.

In the process of the present invention it is preferable to use those starting materials and intermediates, in each case in free form or in salt form, which result in the compounds of formula (I) and their salts described at the beginning as being especially valuable.

In the area of pest control, the compounds of formula (I) according to the invention are active ingredients exhibiting valuable preventive and/or curative activity with a very advantageous biocidal spectrum and a very broad spectrum, even at low rates of concentration, while being well tolerated by warm-blooded animals, fish and plants. They are, surprisingly, equally suitable for controlling both plant pests and ecto- and endo-parasites in humans and more especially in productive livestock, domestic animals and pets. They are effective against all or individual development stages of normally sensitive animal pests, but also of resistant animal pests, such as insects and representatives of the order Acarina, nematodes, cestodes and trematodes, while at the same time protecting useful organisms. The insecticidal or acaricidal activity of the active ingredients according to the invention may manifest itself directly, i.e. in the mortality of the pests, which occurs immediately or only after some time, for example during moulting, or indirectly, for example in reduced oviposition and/or hatching rate. Good activity corresponds to a mortality of at least 50 to 60 %.

Successful control within the scope of the subject of the invention is possible, in particular, of pests from the orders Lepidoptera, Coleoptera, Orthoptera, lsoptera, Psocoptera, Anoplura, Mallophaga, Thysanoptera, Heteroptera, Homoptera, Hymenoptera, Diptera, Siphonaptera, Thysanura and Acarina, mainly Acarina, Diptera, Thysanoptera, Lepidoptera and Coleoptera. Very especially good control is possible of the following pests:
Abagrotis spp., Abraxas spp., Acantholeucania spp., Acanthoplusia spp., Acarus spp., Acarus siro, Aceria spp., Aceria sheldoni, Acleris spp., Acoloithus spp., Acompsia spp., Acossus spp., Acria spp., Acrobasis spp., Acrocercops spp., Acrolepia spp., Acrolepiopsis spp., Acronicta spp., Acropolitis spp., Actebia spp., Aculus spp., Aculus schlechtendali, Adoxophyes spp., Adoxophyes reticulana, Aedes spp., Aegeria spp., Aethes spp., Agapeta spp., Agonopterix spp., Agriopis spp., Agriotes spp., Agriphila spp., Agrochola spp., Agroperina spp., Alabama spp., Alabama argillaceae, Agrotis spp., Albuna spp., Alcathoe spp., Alcis spp., Aleimma spp., Aletia spp., Aleurothrixus spp., Aleurothrixus floccosus, Aleyrodes spp., Aleyrodes brassicae, Allophyes spp., Alsophila spp., Amata spp., Amathes spp., Amblyomma spp., Amblyptilia spp., Ammoconia spp., Amorbia spp., Amphion spp., Amphipoea spp., Amphipyra spp., Amyelois spp., Anacamptodes spp., Anagrapha spp., Anarsia spp., Anatrychyntis spp., Anavitrinella spp., Ancylis spp., Andropolia spp., Anhimella spp., Antheraea spp., Antherigona spp., Antherigona soccata, Anthonomus spp., Anthonomus grandis, Anticarsia spp., Anticarsia gemmatalis, Aonidiella spp., Apamea spp., Aphania spp., Aphelia spp., Aphididae, Aphis spp., Apotomis spp., Aproaerema spp., Archippus spp., Archips spp., Acromyrmex, Arctia spp., Argas spp., Argolamprotes spp., Argyresthia spp., Argyrogramma spp., Argyroploce spp., Argyrotaenia spp., Arotrophora spp., Ascotis spp., Aspidiotus spp., Aspilapteryx spp., Asthenoptycha spp., Aterpia spp., Athetis spp., Atomaria spp., Atomaria linearis, Atta spp., Atypha spp., Autographa spp., Axylia spp., Bactra spp., Barbara spp., Batrachedra spp., Battaristis spp., Bembecia spp., Bemisia spp., Bemisia tabaci, Bibio spp., Bibio hortulanis, Bisigna spp., Blastesthia spp., Blatta spp., Blatella spp., Blepharosis spp., Bleptina spp., Boarmia spp., Bombyx spp., Bomolocha spp., Boophilus spp., Brachmia spp., Bradina spp., Brevipalpus spp., Brithys spp., Bryobia spp., Bryobia praetiosa, Bryotropha spp., Bupalus spp., Busseola spp., Busseola fusca, Cabera spp., Cacoecimorpha spp., Cadra spp., Cadra cautella, Gaenurgina spp., Calipitrimerus spp., Callierges spp., Callophpora spp., Callophpora erythrocephala, Calophasia spp., Caloptilia spp., Calybites spp., Capnoptycha spp., Capua spp., Caradrina spp., Caripeta spp., Carmenta spp., Carposina spp., Carposina nipponensis, Catamacta spp., Catelaphris spp., Catoptria spp., Caustoloma spp., Celaena spp., Celypha spp., Cenopis spp., Cephus spp., Ceramica spp., Cerapteryx spp., Ceratitis spp, Ceratophyllus spp., Ceroplaster spp., Chaetocnema spp., Chaetocnema tibialis, Chamaesphecia spp., Charanvca spp., Cheimophila spp., Chersotis spp., Chiasmia spp., Chilo spp., Chionodes spp., Chorioptes spp., Choristoneura spp., Chrysaspidia spp., Chrysodeixis spp., Chrysomya spp., Chrysomphalus spp., Chrysomphalus dictyospermi, Chrysomphalus aonidium, Chrysoteuchia spp., Cilix spp., Cimex spp., Clysia spp., Clysia ambiguella, Clepsis spp., Cnaemidophorus spp., Cnaphalocrocis spp., Cnephasia spp., Coccus spp., Coccus hesperidum, Cochylis spp., Coleophora spp., Colotois spp., Commophila spp., Conistra spp., Conopomorpha spp., Corcyra spp., Cornutiplusia spp., Cosmia spp., Cosmopolites spp., Cosmopterix spp., Cossus spp., Costaeonvexa spp., Crambus spp., Creatonotos spp., Crocidolomia spp., Crocidolomia binotalis, Croesia spp., Crymodes spp., Cryptaspasma spp., Cryptoblabes spp., Cryptocala spp., Cryptophlebia spp., Cryptophlebia leucotreta, Cryptoptila spp., Ctenopseustis spp., Ctenocephalides spp., Cucullia spp., Curculio spp., Culex spp., Cuterebra spp., Cydia spp., Cydia pomonella, Cymbalophora spp., Dactylethra spp., Dacus spp., Dadica spp., Damalinea spp., Dasychira spp., Decadarchis spp., Decodes spp., Deilephila spp., Deltodes spp., Dendrolimus spp., Depressaria spp., Dermestes spp., Dermanyssus spp., Dermanyssus gallinae, Diabrotica spp., Diachrysia spp., Diaphania spp., Diarsia spp., Diasemia spp., Diatraea spp., Diceratura spp., Dichomeris spp., Dichrocrocis spp., Dichrorampha spp., Dicycla spp., Dioryctria spp., Diparopsis spp., Diparopsis castanea, Dipleurina spp., Diprion spp., Diprionidae, Discestra spp., Distantiella spp., Distantiella theobroma, Ditula spp., Diurnea spp., Doratopteryx spp., Drepana spp., Drosphila spp., Drosphila melanogaster, Dysauxes spp., Dysdercus spp., Dysstroma spp., Eana spp., Earias spp., Ecclitica spp., Ecdytolopha spp., Ecpyrrhorrhoe spp., Ectomyelois spp., Eetropis spp., Egira spp., Elasmopalpus spp., Emmelia spp., mpoasca spp., Empyreuma spp., Enargia spp., Enarmonia spp., Endopiza spp., Endothenia spp., Endotricha spp., Eoreuma spp., Eotetranychus spp., Eotetranychus carpini, Epagoge spp., Epelis spp., Ephestia spp., Ephestiodes spp., Epiblema spp., Epiehoristodes spp., Epinotia spp., Epiphyas spp., Epiplema spp., Epipsestis spp., Epirrhoe spp., Episimus spp., Epitymbia spp., Epllachna spp., Erannis spp., Erastria spp., Eremnus spp., Ereunetis spp., Eriophyes spp., Eriosoma spp., Eriosoma lanigerum, Erythroneura spp., Estigmene spp., Ethmia spp., Etiella spp., Euagrotis spp., Eucosma spp., Euehlaena spp., Euelidia spp., Eueosma spp., Euchistus spp., Eucosmomorpha spp., Eudonia spp., Eufidonia spp., Euhyponomeutoides spp., Eulepitodes spp., Eulia spp., Eulithis spp., Eupithecia spp., Euplexia spp., Eupoecilia spp., Eupoecilia ambiguella, Euproctis spp., Eupsilia spp., Eurhodope spp., Eurois spp., Eurygaster spp., Eurythmia spp., Eustrotia spp., Euxoa spp., Euzophera spp., Evergestis spp., Evippe spp., Exartema spp., Fannia spp., Faronta spp., Feltia spp., Filatima spp., Fishia spp., Frankliniella spp., Fumibotys spp., Gaesa spp., Gasgardia spp., Gastrophilus spp., Gelechia spp., Gilpinia spp., Gilpinia polytoma, Glossina spp., Glyphipterix spp., Glyphodes spp., Gnorimoschemini spp., Gonodonta spp., Gortyna spp., Gracillaria spp., Graphania spp., Grapholita spp., Grapholitha spp., Gravitarmata spp., Gretchena spp., Griselda spp., Gryllotalpa spp., Gynaephora spp., Gypsonoma spp., Hada spp., Haematopinus spp., Halisidota spp., Harpipteryx spp., Harrisina spp., Hedya spp., Helicoverpa spp., Heliophobus spp., Heliothis spp., Hellula spp., Helotropa spp., Hemaris spp., Hercinothrips spp., Herculia spp., Hermonassa spp., Heterogenea spp., Holomelina spp., Homadaula spp., Homoeosoma spp., Homoglaea spp., Homohadena spp., Homona spp., Homonopsis spp., Hoplocampa spp., Hoplodrina spp., Hoshinoa spp., Hxalomma spp., Hydraecia spp., Hydriomena spp., Hyles spp., Hyloicus spp., Hypagyrtis spp., Hypatima spp., Hyphantria spp., Hyphantria cunea, Hypocala spp., Hypocoena spp., Hypodema spp., Hyppobosca spp., Hypsipyla spp., Hyssia spp., Hysterosia spp., Idaea spp., Idia spp., Ipimorpha spp., Isia spp., Isochorista spp., Isophrictis spp., Isopolia spp., lsotrias spp., Ixodes spp., ltame spp., Jodia spp., Jodis spp., Kawabea spp., Keiferia spp., Keiferia lycopersicella, Labdia spp., Lacinipolia spp., Lambdina spp., Lamprothritpa spp., Laodelphax spp., Lasius spp., Laspeyresia spp., Leptinotarsa spp., Leptinotarsa decemlineata, Leptocorisa spp., Leptostales spp., Lecanium spp., Lecanium comi, Lepidosaphes spp., Lepisma spp., Lepisma saccharina , Lesmone spp., Leucania spp., Leucinodes spp., Leucophaea spp., Leucophaea maderae, Leucoptera spp., Leucoptera scitella, Linognathus spp., Liposcelis spp., Lissorhoptrus spp., Lithacodia spp., Lithocolletis spp., Lithomoia spp., Lithophane spp., Lixodessa spp., Lobesia spp., Lobesia botrana, Lobophora spp., Locusta spp., Lomanaltes spp., Lomographa spp., Loxagrotis spp., Loxostege spp., Lucilia spp., Lymantria spp., Lymnaecia spp., Lyonetia spp., Lyriomyza spp., Macdonnoughia spp., Macrauzata spp., Macronoctua spp., Macrosiphus spp., Malacosoma spp., Maliarpha spp., Mamestra spp., Mamestra brassicae, Manduca spp., Manduca sexta, Marasmia spp., Margaritia spp., Matratinea spp., Matsumuraeses spp., Melanagromyza spp., Melipotes spp., Melissopus spp., Melittia spp., Melolontha spp., Meristis spp., Meritastis spp., Merophyas spp., Mesapamea spp., Mesogona spp., Mesoleuca spp., Metanema spp., Metendothenia spp., Metzneria spp., Micardia spp., Microcorses spp., Microleon spp., Mnesictena spp., Mocis spp., Monima spp., Monochroa spp., Monomorium spp., Monomorium pharaonis, Monopsis spp., Morrisonia spp., Musca spp., Mutuuraia spp., Myelois spp., Mythimna spp., Myzus spp., Naranga spp., Nedra spp., Nemapogon spp., Neodiprion spp., Neosphaleroptera spp., Nephelodes spp., Nephotettix spp., Nezara spp., Nilaparvata spp., Niphonympha spp., Nippoptilia spp., Noctua spp., Nola spp., Notocelia spp., Notodonta spp., Nudaurelia spp., Ochropleura spp., Ocnerostoma spp., Oestrus spp., Olethreutes spp., Oligia spp., Olindia spp., Olygonychus spp., Olygonychus gallinae, Oncocnemis spp., Operophtera spp., Ophisma spp., Opogona spp., Oraesia spp., Orniodoros spp., Orgyia spp., Oria spp., Orseolia spp., Orthodes spp., Orthogonia spp., Orthosia spp., Oryzaephilus spp., Oscinella spp., Oscinella frit, Osminia spp., Ostrinia spp., Ostrinia nubilalis, Otiorhynchus spp., Ourapteryx spp., Pachetra spp., Pachysphinx spp., Pagyda spp., Paleacrita spp., Paliga spp., Palthis spp., Pammene spp., Pandemis spp., Panemeria spp., Panolis spp., Panolis flammea, Panonychus spp., Parargyresthia spp., Paradiarsia spp., Paralobesia spp., Paranthrene spp., Parapandemis spp., Parapediasia spp., Parastichtis spp., Parasyndemis spp., Paratoria spp., Pareromeme spp., Pectinophora spp., Pectinophora gossypiella, Pediculus spp., Pegomyia spp., Pegomyia hyoscyami, Pelochrista spp., Pennisetia spp., Penstemonia spp., Pemphigus spp., Peribatodes spp., Peridroma spp., Perileucoptera spp., Periplaneta spp., Perizoma spp., Petrova spp., Pexicopia spp., Phalonia spp., Phalonidia spp., Phaneta spp., Phlyctaenia spp., Phlyctinus spp., Phorbia spp., Phragmatobia spp., Phricanthes spp., Phthorimaea spp., Phthorimaea operculella, Phyllocnistis spp., Phyllocoptruta spp., Phyllocoptruta oleivora, Phyllonorycter spp., Phyllophila spp., Phylloxera spp., Pieris spp., Pieris rapae, Piesma spp., Planococus spp., Planotortrix spp., Platyedra spp., Platynota spp., Platyptilia spp., Platysenta spp., Plodia spp., Plusia spp., Plutella spp., Plutella xylostella, Podosesia spp., Polia spp., Popillia spp., Polymixis spp., Polyphagotarsonemus spp., Polyphagotarsonemus latus, Prays spp., Prionoxystus spp., Probole spp., Proceras spp., Prochoerodes spp., Proeulia spp., Proschistis spp., Proselena spp., Proserpinus spp., Protagrotis spp., Proteoteras spp., Protobathra spp., Protoschinia spp., Pselnophorus spp., Pseudaletia spp., Pseudanthonomus spp., Pseudaternelia spp., Pseudaulacaspis spp., Pseudexentera spp., Pseudococus spp., Pseudohermenias spp., Pseudoplusia spp., Psoroptes spp., Psylla spp., Psylliodes spp., Pterophorus spp., Ptycholoma spp., Pulvinaria spp., Pulvinaria aethiopica, Pyralis spp., Pyrausta spp., Pyrgotis spp., Pyrreferra spp., Pyrrharctia spp., Quadraspidiotus spp., Rancora spp., Raphia spp., Reticultermes spp., Retinia spp., Rhagoletis spp, Rhagoletis pomonella, Rhipicephalus spp., Rhizoglyphus spp., Rhizopertha spp., Rhodnius spp., Rhophalosiphum spp., Rhopobota spp., Rhyacia spp., Rhyacionia spp., Rhynchopacha spp., Rhyzosthenes spp., Rivula spp., Rondotia spp., Rusidrina spp., Rynchaglaea spp., Sabulodes spp., Sahlbergella spp., Sahlbergella singularis, Saissetia spp., Samia spp., Sannina spp., Sanninoidea spp., Saphoideus spp., Sarcoptes spp., Sathrobrota spp., Scarabeidae, Sceliodes spp., Schinia spp., Schistocerca spp., Schizaphis spp., Schizura spp., Schreckensteinia spp., Sciara spp., Scirpophaga spp., Scirthrips auranti, Scoparia spp., Scopula spp., Scotia spp., Scotinophara spp., Scotogramma spp., Scrobipalpa spp., Scrobipalpopsis spp., Semiothisa spp., Sereda spp., Sesamia spp., Sesia spp., Sicya spp., Sideridis spp., Simyra spp., Sineugraphe spp., Sitochroa spp., Sitobion spp., Sitophilus spp., Sitotroga spp., Solenopsis spp., Smerinthus spp., Sophronia spp., Spaelotis spp., Spargaloma spp., Sparganothis spp., Spatalistis spp., Sperchia spp., Sphecia spp., Sphinx spp., Spilonota spp., Spodoptera spp., Spodoptera littoralis, Stagmatophora spp., Staphylinochrous spp., Stathmopoda spp., Stenodes spp., Sterrha spp., Stomoxys spp., Strophedra spp., Sunira spp., Sutyna spp., Swammerdamia spp., Syllomatia spp., Sympistis spp., Synanthedon spp., Synaxis spp., Syncopacma spp., Syndemis spp., Syngrapha spp., Synthomeida spp., Tabanus spp., Taeniarchis spp., Taeniothrips spp., Tannia spp., Tarsonemus spp., Tegulifera spp., Tehama spp., Teleiodes spp., Telorta spp., Tenebrio spp., Tephrina spp., Teratoglaea spp., Terricula spp., Tethea spp., Tetranychus spp., Thalpophila spp., Thaumetopoea spp., Thiodia spp., Thrips spp., Thrips palmi, Thrips tabaci, Thyridopteryx spp., Thyris spp., Tineola spp., Tipula spp., Tortricidia spp., Tortrix spp., Trachea spp., Trialeurodes spp., Trialeurodes vaporariorum, Triatoma spp., Triaxomera spp., Tribolium spp., Tricodectes spp., Trichoplusia spp., Trichoplusia ni, Trichoptilus spp., Trioza spp., Trioza erytreae, Triphaenia spp., Triphosa spp., Trogoderma spp., Tyria spp., Udea spp., Unaspis spp., Unaspis citri, Utetheisa spp., Valeriodes spp., Vespa spp., Vespamima spp., Vitacea spp., Vitula spp., Witlesia spp., Xanthia spp., Xanthorhoe spp., Xanthotype spp., Xenomicta spp., Xenopsylla spp., Xenopsylla cheopsis, Xestia spp., Xylena spp., Xylomyges spp., Xyrosaris spp., Yponomeuta spp., Ypsolopha spp., Zale spp., Zanclognathus spp., Zeiraphera spp., Zenodoxus spp., Zeuzera spp., Zygaena spp.,

It is also possible to control pests of the class Nematoda using the compounds according to the invention. Such pests include, for example,
root knot nematodes, cyst-forming nematodes and also stem and leaf nematodes;
especially of Heterodera spp., e.g. Heterodera schachtii, Heterodora avenae and Heterodora trifolii; Globodera spp., e.g. Globodera rostochiensis; Meloidogyne spp., e.g. Meloidogyne incognita and Meloidogyne javanica; Radopholus spp., e.g. Radopholus similis; Pratylenchus, e.g. Pratylenchus neglectans and Pratylenchus penetrans; Tylenchulus, e.g. Tylenchulus semipenetrans; Longidorus, Trichodorus, Xiphinema, Ditylenchus, Apheenchoides and Anguina; especially Meloidogyne, e.g. Meloidogyne incognita, and Heterodera, e.g. Heterodera glycines.

An especially important aspect of the present invention is the use of the compounds of formula (I) according to the invention in the protection of plants against parasitic feeding pests.

The action of the compounds according to the invention and the compositions comprising them against animal pests can be significantly broadened and adapted to the given circumstances by the addition of other insecticides; acaricides or nematicides. Suitable additives include, for example, representatives of the following classes of active ingredient: organophosphorus compounds, nitrophenols and derivatives, formamidines, ureas, carbamates, pyrethroids, chlorinated hydrocarbons, neonicotinoids and Bacillus thuringiensis preparations.

Examples of especially suitable mixing partners include: azamethiphos; chlorfenvinphos; cypermethrin, cypermethrin high-cis; cyromazine; diafenthiuron; diazinon; dichlorvos; dicrotophos; dicyclanil; fenoxycarb; fluazuron; furathiocarb; isazofos; iodfenphos; kinoprene; lufenuron; methacriphos; methidathion; monocrotophos; phosphamidon; profenofos; diofenolan; a compound obtainable from the Bacillus thuringiensis strain GC91 or from strain NCTC11821; pymetrozine; bromopropylate; methoprene; disulfoton; quinalphos; taufluvalinate; thiocyclam; thiometon; aldicarb; azinphos-methyl; benfuracarb; bifenthrin; buprofezin; carbofuran; dibutylaminothio; cartap; chlorfluazuron; chlorpyrifos; clothianidin; cyfluthrin; lambda-cyhalothrin; alpha-cypermethrin; zeta-cypermethrin; deltamethrin; diflubenzuron; endosulfan; ethiofencarb; fenitrothion; fenobucarb; fenvalerate; formothion; methiocarb; heptenophos; imidacloprid; isoprocarb; methamidophos; methomyl; mevinphos; parathion; parathion-methyl; phosalone; pirimicarb; propoxur; teflubenzuron; terbufos; triazamate; fenobucarb; tebufenozide; fipronil; beta-cyfluthrin; silafluofen; fenpyroximate; pyridaben; pyridalyl; fenazaquin; pyriproxyfen; pyrimidifen; nitenpyram; acetamiprid; emamectin; emamectin-benzoate; spinosad; a plant extract that is active against insects; a preparation that comprises nematodes and is active against insects; a preparation obtainable from Bacillus subtilis; a preparation that comprises fungi and is active against insects; a preparation that comprises viruses and is active against insects; chlorfenapyr; acephate; acrinathrin; alanycarb; alphamethrin; amitraz; AZ 60541; azinphos A; azinphos M; azocyclotin; bendiocarb; bensultap; beta-cyfluthrin; brofenprox; bromophos A; bufencarb; butocarboxin; butylpyridaben; cadusafos; carbaryl; carbophenothion; chloethocarb; chlorethoxyfos; chlormephos; cis-resmethrin; clocythrin; clofentezine; cyanophos; cycloprothrin; cyhexatin; demeton M; demeton S; demeton-S-methyl; dichlofenthion; dicliphos; diethion; dimethoate; dimethylvinphos; dioxathion; edifenphos; esfenvalerate; ethion; ethofenprox; ethoprophos; etrimphos; fenamiphos; fenbutatin oxide; fenothiocarb; fenpropathrin; fenpyrad; fenthion; fluazinam; flucycloxuron; flucythrinate; flufenoxuron; flufenprox; fonophos; fosthiazate; fubfenprox; HCH; hexaflumuron; hexythiazox; flonicamid; iprobenfos; isofenphos; isoxathion; ivermectin; malathion; mecarbam; mesulfenphos; metaldehyde; metolcarb; milbemectin; moxidectin; naled; NC 184; nithiazine; omethoate; oxamyl; oxydemethon M; oxydeprofos; permethrin; phenthoate; phorate; phosmet; phoxim; pirimiphos M; pirimiphos E; promecarb; propaphos; prothiofos; prothoate; pyrachlophos; pyradaphenthion; pyresmethrin; pyrethrum; tebufenozide; salithion; sebufos; sulfotep; sulprofos; tebufenpyrad; tebupirimphos; tefluthrin; temephos; terbam; tetrachlorvinphos; thiacloprid; thiafenox; thiamethoxam; thiodicarb; thiofanox; thionazin; thuringiensin; tralomethrin; triarathene; triazophos; triazuron; trichlorfon; triflumuron; trimethacarb; vamidothion; xylylcarb; etoxazole; zetamethrin; indoxacarb; methoxyfenozide; bifenazate; XMC (3,5-xylyl methylcarbamate); or the fungus pathogen Metarhizium anisopliae.

The compounds according to the invention can be used to control, i.e. to inhibit or destroy, pests of the mentioned type occurring on plants, especially on useful plants and ornamentals in agriculture, in horticulture and in forestry, or on parts of such plants, such as the fruits, blossoms, leaves, stems, tubers or roots, while in some cases plant parts that grow later are still protected against those pests.

Target crops include especially cereals, such as wheat, barley, rye, oats, rice, maize and sorghum; beet, such as sugar beet and fodder beet; fruit, e.g. pomes, stone fruit and soft fruit, such as apples, pears, plums, peaches, almonds, cherries and berries, e.g. strawberries, raspberries and blackberries; leguminous plants, such as beans, lentils, peas and soybeans; oil plants, such as rape, mustard, poppy, olives, sunflowers, coconut, castor oil, cocoa and groundnuts; cucurbitaceae, such as marrows, cucumbers and melons; fibre plants, such as cotton, flax, hemp and jute; citrus fruits, such as oranges, lemons, grapefruit and mandarins; vegetables, such as spinach, lettuce, asparagus, cabbages, carrots, onions, tomatoes, potatoes and paprika; lauraceae, such as avocado, cinnamon and camphor; and tobacco, nuts, coffee, aubergines, sugar cane, tea, pepper, vines, hops, bananas, natural rubber plants and ornamentals.

Further areas of use of the compounds according to the invention are the protection of stored goods and storerooms and the protection of raw materials, and also in the hygiene sector, especially the protection of domestic animals and productive livestock against pests of the mentioned type, more especially the protection of domestic animals, especially cats and dogs, from infestation by fleas, ticks and nematodes.

The invention therefore relates also to pesticidal compositions, such as emulsifiable concentrates, suspension concentrates, directly sprayable or dilutable solutions, spreadable pastes, dilute emulsions, wettable powders, soluble powders, dispersible powders, wettable powders, dusts, granules and encapsulations of polymer substances, that comprise at least one of the compounds according to the invention, the choice of formulation being made in accordance with the intended objectives and the prevailing circumstances.

The active ingredient is used in those compositions in pure form, a solid active ingredient, for example, in a specific particle size, or preferably together with at least one of the adjuvants customary in formulation technology, such as extenders, e.g. solvents or solid carriers, or surface-active compounds (surfactants). In the area of parasite control in humans, domestic animals, productive livestock and pets it will be self-evident that only physiologically tolerable additives are used.

Solvents are, for example: non-hydrogenated or partly hydrogenated aromatic hydrocarbons, preferably fractions C₈ to C₁₂ of alkylbenzenes, such as xylene mixtures, alkylated naphthalenes or tetrahydronaphthalene, aliphatic or cycloaliphatic hydrocarbons, such as paraffins or cyclohexane, alcohols, such as ethanol, propanol or butanol, glycols and ethers and esters thereof, such as propylene glycol, dipropylene glycol ether, ethylene glycol or ethylene glycol monomethyl or -ethyl ether, ketones, such as cyclohexanone, isophorone or diacetone alcohol, strongly polar solvents, such as N-methylpyrrolid-2-one, dimethyl sulfoxide or N,N-dimethylformamide, water, non-epoxidized or epoxidized plant oils, such as non-epoxidized or epoxidized rapeseed, castor, coconut or soya oil, and silicone oils.

The solid carriers used, for example for dusts and dispersible powders, are as a rule natural rock powders, such as calcite, talc, kaolin, montmorillonite or attapulgite. Highly disperse silicic acids or highly disperse absorbent polymers can also be added to improve the physical properties. Granular adsorptive granule carriers are porous types, such as pumice, crushed brick, sepiolite or bentonite, and non-sorbent carrier materials are calcite or sand. A large number of granular materials of inorganic or organic nature can furthermore be used, in particular dolomite or comminuted plant residues.

Surface-active compounds are, depending on the nature of the active compound to be formulated, nonionic, cationic and/or anionic surfactants or surfactant mixtures with good emulsifying, dispersing and wetting properties. The surfactants listed below are to be regarded only as examples; many other surfactants which are customary in formulation technology and are suitable according to the invention are described in the relevant literature.

Nonionic surfactants are, in particular, polyglycol ether derivatives of aliphatic or cycloaliphatic alcohols, saturated or unsaturated fatty acids and alkylphenols, which can contain 3 to 30 glycol ether groups and 8 to 20 carbon atoms in the (aliphatic) hydrocarbon radical and 6 to 18 carbon atoms in the alkyl radical of the alkylphenols. Substances which are furthermore suitable are water-soluble polyethylene oxide adducts, containing 20 to 250 ethylene glycol ether and 10 to 100 propylene glycol ether groups, on propylene glycol, ethylene diaminopolypropylene glycol and alkyl polypropylene glycol having 1 to 10 carbon atoms in the alkyl chain. The compounds mentioned usually contain 1 to 5 ethylene glycol units per propylene glycol unit. Examples are nonylphenol-polyethoxyethanols, castor oil polyglycol ethers, polypropylene-polyethylene oxide adducts, tributylphenoxypolyethoxyethanol, polyethylene glycol and octylphenoxypolyethoxyethanol. Other substances are fatty acid esters of polyoxyethylene sorbitan, such as polyoxyethylene sorbitan trioleate.

The cationic surfactants are, in particular, quaternary ammonium salts which contain, as substituents, at least one alkyl radical having 8 to 22 C atoms and, as further substituents, lower, non-halogenated or halogenated alkyl, benzyl or lower hydroxyalkyl radicals. The salts are preferably in the form of halides, methyl-sulfates or ethyl-sulfates. Examples are stearyl-trimethyl-ammonium chloride and benzyl-di-(2-chloroethyl)-ethylammonium bromide.

Suitable anionic surfactants can be both water-soluble soaps and water-soluble synthetic surface-active compounds. Suitable soaps are the alkali metal, alkaline earth metal and substituted or unsubstituted ammonium salts of higher fatty acids (C₁₀-C₂₂), such as the sodium or potassium salts of oleic or stearic acid, or of naturally occurring fatty acid mixtures, which can be obtained, for example, from coconut oil or tall oil; and furthermore also the fatty acid methyl-taurine salts. However, synthetic surfactants are more frequently used, in particular fatty sulfonates, fatty sulfates, sulfonated benzimidazole derivatives or alkylarylsulfonates. The fatty sulfonates and sulfates are as a rule in the form of alkali metal, alkaline earth metal or substituted or unsubstituted ammonium salts and in general have an alkyl radical of 8 to 22 C atoms, alkyl also including the alkyl moiety of acyl radicals; examples are the sodium or calcium salt of ligninsulfonic acid, of dodecylsulfuric acid ester or of a fatty alcohol sulfate mixture prepared from naturally occurring fatty acids. These also include the salts of sulfuric acid esters and sulfonic acids of fatty alcohol-ethylene oxide adducts. The sulfonated benzimidazole derivatives preferably contain 2 sulfonic acid groups and a fatty acid radical having about 8 to 22 C atoms. Alkylarylsulfonates are, for example, the sodium, calcium or triethanolammonium salts of dodecylbenzenesulfonic acid, of dibutylnaphthalenesulfonic acid or of a naphthalenesulfonic acid-formaldehyde condensation product. Corresponding phosphates, such as salts of the phosphoric acid ester of a p-nonylphenol-(4-14)-ethylene oxide adduct or phospholipids, can further also be used.

The compositions as a rule comprise 0.1 to 99 %, in particular 0.1 to 95 %, of active compound and 1 to 99.9 %, in particular 5 to 99.9 %, of - at least - one solid or liquid auxiliary, it being possible as a rule for 0 to 25 %, in particular 0.1 to 20 %, of the composition to be surfactants (% is in each case per cent by weight). While concentrated compositions are more preferred as commercial goods, the end user as a rule uses dilute compositions which comprise considerably lower concentrations of active compound. Preferred compositions are composed, in particular, as follows (% = per cent by weight):

| Emulsifiable concentrates: | |
|---|---|
| active ingredient: | 1 to 90%, preferably 5 to 20% |
| surfactant: | 1 to 30%, preferably 10 to 20% |
| solvent: | 5 to 98%, preferably 70 to 85% |

| Dusts: | |
|---|---|
| active ingredient: | 0.1 to 10%, preferably 0.1 to 1% |
| solid carrier: | 99.9 to 90%, preferably 99.9 to 99% |

| Suspension concentrates: | |
|---|---|
| active ingredient: | 5 to 75%, preferably 10 to 50% |
| water: | 94 to 24%, preferably 88 to 30% |
| surfactant: | 1 to 40%, preferably 2 to 30% |

| Wettable powders: | |
|---|---|
| active ingredient: | 0.5 to 90%, preferably 1 to 80% |
| surfactant: | 0.5 to 20%, preferably 1 to 15% |
| solid carrier: | 5 to 99%, preferably 15 to 98% |

| Granules: | |
|---|---|
| active ingredient: | 0.5 to 30%, preferably 3 to 15% |
| solid carrier: | 99.5 to 70%, preferably 97 to 85% |

The compositions according to the invention may also comprise further solid or liquid adjuvants, such as stabilisers, e.g. vegetable oils or epoxidised vegetable oils (e.g. epoxidised coconut oil, rapeseed oil or soybean oil), antifoams, e.g. silicone oil, preservatives, viscosity regulators, binders and/or tackifiers as well as fertilisers or other active ingredients for obtaining special effects, e.g. acaricides, bactericides, fungicides, nematicides, molluscicides or selective herbicides.

The crop protection products according to the invention are prepared in known manner, in the absence of adjuvants, e.g. by grinding, sieving and/or compressing a solid active ingredient or mixture of active ingredients, for example to a certain particle size, and in the presence of at least one adjuvant, for example by intimately mixing and/or grinding the active ingredient or mixture of active ingredients with the adjuvant(s). The invention relates likewise to those processes for the preparation of the compositions according to the invention and to the use of the compounds of formula (I) in the preparation of those compositions.

The invention relates also to the methods of application of the crop protection products, i.e. the methods of controlling pests of the mentioned type, such as spraying, atomising, dusting, coating, dressing, scattering or pouring, which are selected in accordance with the intended objectives and the prevailing circumstances, and to the use of the compositions for controlling pests of the mentioned type. Typical rates of concentration are from 0.1 to 1000 ppm, preferably from 0.1 to 500 ppm, of active ingredient. The rates of application per hectare are generally from 1 to 2000 g of active ingredient per hectare, especially from 10 to 1000 g/ha, preferably from 20 to 600 g/ha.

A preferred method of application in the area of crop protection is application to the foliage of the plants (foliar application), the frequency and the rate of application being dependent upon the risk of infestation by the pest in question. However, the active ingredient can also penetrate the plants through the roots (systemic action) when the locus of the plants is impregnated with a liquid formulation or when the active ingredient is incorporated in solid form into the locus of the plants, for example into the soil, e.g. in granular form (soil application). In the case of paddy rice crops, such granules may be applied in metered amounts to the flooded rice field.

The crop protection products according to the invention are also suitable for protecting plant propagation material, e.g. seed, such as fruits, tubers or grains, or plant cuttings, against animal pests. The propagation material can be treated with the composition before planting: seed, for example, can be dressed before being sown. The active ingredients according to the invention can also be applied to grains (coating), either by impregnating the seeds in a liquid formulation or by coating them with a solid formulation. The composition can also be applied to the planting site when the propagation material is being planted, for example to the seed furrow during sowing. The invention relates also to such methods of treating plant propagation material and to the plant propagation material so treated.

The following Examples serve to illustrate the invention. They do not limit the invention. Temperatures are given in degrees Celsius; mixing ratios of solvents are given in parts by volume.

### Preparation Examples:

### Example P1): Preparation of 3-(4-{3-[2,6-dichloro-4-(3,3-dichloro-allyloxy)-phenoxyl propoxyl-phenyl)-acrylonitrile

58 mg of cyanomethanephosphoric acid diethyl ester, 59 mg of 5.4 molar solution of sodium methoxide in methanol and 150 mg of 4-13-[2,6-dichloro-4-(3,3-dichloro-allyloxy)-phenoxy]-propoxy}-benzaldehyde are stirred for 24 hours at room temperature in 2 ml of ethanol. The reaction mixture is poured into water and extracted with ethyl acetate. Concentration of the organic phase and purification over silica gel yield the title compound (compound 1.1).

### Example P2): Preparation of 4-(4-{3-[2,6-dichloro-4-(3,3-dichloro-allyloxy)-phenoxy]-propoxy}-phenyl)-but-3-yn-2-ol

P2.1. 740 mg of tetrakis(triphenylphosphine)palladium and 427 mg of copper(I) iodide are stirred for 5 minutes in 25 ml of diisopropylamine. 7 g of 4-iodophenol and a solution of 2.2 g of 3-butyn-2-ol in 25 ml of diisopropylamine are then added. After 4 hours' stirring at room temperature, the reaction mixture is poured into ammonium chloride solution and extracted with ethyl acetate. Concentration of the organic phase and purification over silica gel yield 4-(3-hydroxy-but-1-ynyl)-phenol. ¹H-NMR (CDCl₃) 300MHz: 1.54 (d,3H), 2.04 (s,1 H), 4.76 (m,1 H), 5.49 (s,1 H), 6.77 (d,2H), 7.30 (d,2H).

P2.2: 840 mg of 4-(3-hydroxy-but-1-ynyl)-phenol, 2.2 g of methanesulfonic acid 3-[2,6-dichloro-4-(3,3-dichloro-allyloxy)-phenoxy]-propyl ester and 2.2 g of potassium carbonate are stirred for 24 hours at 50°C in 20 ml of dimethylformamide. The reaction mixture is poured onto water and extracted with ethyl acetate. Concentration of the organic phase and purification over silica gel yield the title compound (compound 1.7).

### Example P3): Preparation of 1,3-dichloro-5-(3,3-dichloro-allyloxy)-2-{3-[4-(3-methoxy-methoxy-but-1-ynyl)-phenoxy]-propoxy}-benzene

20 mg of chloromethyl methyl ether and 32 mg of N-ethyl-diisopropylamine are added to a solution of 100 mg of 4-(4-{3-[2,6-dichloro-4-(3,3-dichloro-allyloxy)-phenoxy]-propoxy}-phenyl)-but-3-yn-2-ol in 4 ml of dichloromethane, and stirring is carried out for 36 hours at room temperature. The reaction mixture is diluted with ethyl acetate and washed in succession with dilute hydrochloric acid and water. Concentration of the organic phase and purification over silica gel yield the title compound (compound 1.11).

### Example P4): Preparation of 4-(4-{3-[2,6-dichloro-4-(3,3-dichloro-allyloxy)-phenoxy]-propoxy}-phenyl)-but-3-yn-2-one

306 mg of dimethyl sulfoxide are added dropwise in the course of 5 minutes at -60°C to a solution of 228 mg of oxalyl chloride in 20 ml of dichloromethane. After 30 minutes, a solution of 800 mg of 4-(4-{3-[2,6-dichloro-4-(3,3-dichloro-allyfoxy)-phenoxy]-propoxy)-phenyl)-but-3-yn-2-ol in 10 ml of dichloromethane is added dropwise in the course of 20 minutes at -60°C. After a further 30 minutes, 1.1 ml of triethylamine are added dropwise, and stirring is carried out for a further 15 minutes at -60°C. The reaction mixture is heated slowly to room temperature, then poured into water and extracted with dichloromethane. Concentration of the organic phase and purification over silica gel yield the title compound (compound 1.15).

### Example P5): Preparation of 4-(4-{3-[2,6-dichloro-4-(3,3-dichloro-allyloxy)-phenoxyl-propoxyl-phenyl)-but-3-yn-2-one O-methyl-oxime

20.9 mg of O-methylhydroxylamine hydrochloride, 20.5 mg of sodium acetate and 122 mg of 4-(4-(3-[2,6-dichloro-4-(3,3-dichloro-allyloxy)-phenoxy]-propoxy)-phenyl)-but-3-yn-2-one are stirred for 24 hours at room temperature in 4 ml of methanol. The reaction mixture is diluted with ethyl acetate and washed with water. Concentration of the organic phase and purification over silica gel yield the title compound (compound 1.16).

### Example P6): Preparation of 1,3-dichloro-5-(3,3-dichloro-allyloxy)-2-{3-[4-(2-nitrovinyl)-phenoxy]-propoxy}-benzene

100 mg of 4-{3-[2,6-dichloro-4-(3,3-dichloro-allyloxy)-phenoxy]-propoxy}-benzaldehyde, 68 mg of ammonium acetate and 83 mg of nitromethane are stirred for 30 minutes at 120°C in 0.1 ml of acetic acid. The reaction mixture is poured into water and extracted with ethyl acetate. Concentration of the organic phase and purification over silica gel yield the title compound (compound 1.4).

### Example P7): Preparation of 4-(4-{3-[2,6-dichloro-4-(3,3-dichloro-allyloxy)-phenoxyl-propoxy}-phenyl)-but-3-en-2-one

52 mg of 4-(4-hydroxy-phenyl)-but-3-en-2-one, 129 mg of methanesulfonic acid 3-[2,6-dichloro-4-(3,3-dichloro-allyloxy)-phenoxy]-propyl ester and 84 mg of potassium carbonate are stirred for 2 hours at 50°C in 2 ml of dimethylformamide. The reaction mixture is poured into water and extracted with ethyl acetate. Concentration of the organic phase and purification over silica gel yield the title compound (compound 1.19).

### Example P8): Preparation of 4-(4-{3-[2,6-dichloro-4-(3,3-dichloro-allyloxy)-phenoxy]-propoxy)-phenyl)-but-3-en-2-one O-ethyl-oxime

100 mg of 4-(4-{3-[2,6-dichloro-4-(3,3-dichloro-allyloxy)-phenoxy]-propoxy}-phenyl)-but-3-en-2-one, 17 mg of O-methylhydroxylamine hydrochloride and 17 mg of sodium acetate are stirred for 16 hours at room temperature in 10 ml of methanol. The reaction mixture is poured into water and extracted with ethyl acetate. Concentration of the organic phase and purification over silica gel yield the title compound (compound 1.14).

### Example P9): Preparation of 3-(4-{3-[2,6-dichloro-4-(3,3-dichloro-allyloxy)-phenoxyl propoxy)-phenyl)-acrylic acid ethyl ester

315 mg of 4-{3-[2,6-dichloro-4-(3,3-dichloro-allyloxy)-phenoxy]-propoxy}-benzaldehyde, 132 mg of phosphonoacetic acid triethyl ester, 164 mg of N-ethyldiisopropylamine and 65 mg of lithium chloride are stirred for 18 hours at room temperature in 5 ml of dimethylformamide. The reaction mixture is poured into water and extracted with ethyl acetate. Concentration of the organic phase and purification over silica gel yield the title compound (compound 1.22).

### Example P10): Preparation of 3-(4-{3-[2,6-dichloro-4-(3,3-dichloro-allyloxy)-phenoxy]-propoxy}-phenyl)-4-methoxy-but-2-ene-nitrile

P10.1 1.8 g of 2-bromo-1-(4-hydroxy-phenyl)-ethanone in 10 ml of methanol are added dropwise to 16 ml of 5.4M sodium methoxide in methanol and 50 ml of methanol, and stirring is carried out for 30 minutes at 60°C. The reaction mixture is concentrated and the residue is taken up in water and adjusted to pH 1 with concentrated hydrochloric acid. Extraction with ethyl acetate is then carried out, the organic phase is concentrated and the crude product is purified over silica gel. 1-(4-Hydroxy-phenyl)-2-methoxy-ethanone is obtained. ¹H-NMR (CDCl₃) 300MHz: 3.50 (s,3H), 4.68 (s,2H), 6.23 (s,1H), 6.91 (d,2H), 7.90 (d,2H).

P10.2 2.76 g of methanesulfonic acid 3-[2,6-dichloro-4-(3,3-dichloro-allyloxy)-phenoxy]-propyl ester, 1.19 g of 1-(4-hydroxy-phenyl)-2-methoxy-ethanone and 2.7 g of potassium carbonate are stirred for 20 hours at 50°C in 90 ml of dimethylformamide. The reaction mixture is poured into water and extracted with ethyl acetate. Concentration of the organic phase and purification over silica gel yield 1-(4-{3-[2,6-dichloro-4-(3,3-dichloro-allyloxy)-phenoxy]-propoxy}-phenyl)-2-methoxy-ethanone. ¹H-NMR (CDCl₃) 300MHz: 2.31 (m,2H), 3.50 (s,3H), 4.17 (t,2H), 4.37 (t,2H), 4.58 (d,2H), 4.66 (s,2H), 6.10 (t,1 H), 6.82 (s,2H), 6.98 (d,2H), 7.93 (d,2H).

P10.3 300 mg of 1-(4-{3-[2,6-dichloro-4-(3,3-dichloro-allyloxy)-phenoxy]-propoxy}-phenyl)-2-methoxy-ethanone and 108 mg of cyanomethyl-phosphonic acid diethyl ester are dissolved in 3 ml of ethanol. After the addition of 0.11 ml of 5.4M sodium methoxide in methanol, stirring is carried out for 17 hours at room temperature. The reaction mixture is diluted with 16 ml of ethyl acetate and washed twice with water. Concentration of the organic phase and purification over silica gel yield the title compound (compound 1.27).

Example P11): The Examples of the following Tables can also be prepared in a manner analogous to that described above. Some of the compounds listed in the Tables are obtained in the form of E/Z isomeric mixtures, although only one isomer is indicated in the E column. Mp. is the melting point in °C, n_{D}²⁰ the refraction index at 20°C and the wavelength of the sodium D-line. The symbol ........... in the formulae signifies the bond of the fragment E to the remainder of the structure.

**Table 1: Compounds of formula**

| | | |
|---|---|---|
| 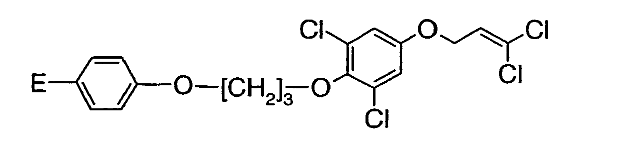 | | |

| No. | E | ¹H-NMR (CDCl₃) 300MHz, melting point or n_{D}²⁰ |
|---|---|---|
| 1.1 | 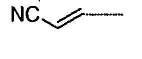 | 2.31 (m,2H), 4.15 (t,2H), 4.31 (t,2H), 4.60 (d,2H), 5.70+5.77(s+s,1H), 6.11 (t,1H), 6.95 (d,2H), 7.38-7.47 (m,2H) |
| 1.2 | 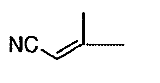 | 2.30 (m,2H), 2.43 (s,3H), 4.16 (t,2H), 4.30 (t,2H), 4.48 (d,2H), 5.55 (s,1H), 6.10 (t,1H), 6.84 (s,1H), 6.94 (d,2H), 7.43 (d,2H) |
| 1.3 | 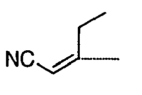 | 1.09+1.15 (t+t,3H), 2.30 (m,2H), 2.59+2.90 (q+q,2H), 4.18 (t,2H), 4.30 (t,2H), 4.59 (d,2H), 5.30+5.44 (s+s,1H), 6.11 (t,1 H), 6.84 (s,2H), 6.92-7.11 (m,2H), 7.40+7.48 (d+d,2H) |
| 1.4 | 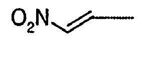 | 2.30 (m,2H), 4.17 (t,2H); 4.34 (t,2H), 4.59 (d,2H), 6.11 (t,1H), 6.81 (s,2H), 6.98 (d,2H), 7.46-7.58 (m,3H), 8.00 (d,1 H) |
| 1.5 | 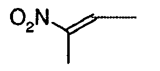 | 2.32 (m,2H), 2.48 (s,3H), 4.17 (t,2H); 4.32 (t,2H), 4.59 (d,2H), 6.10 (t,1H), 6.84 (s,2H), 7.00 (d,2H), 7.43 (d,2H), 8.09 (s,1H) |
| 1.6 | 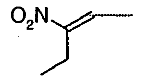 | 1.30 (t,3H), 2.32 (m,2H), 2.91 (q,2H), 4.17 (t,2H); 4.34 (t,2H), 4.59 (d,2H), 6.10 (t,1 H), 6.85 (s,2H), 7.00 (d,2H), 7.42 (d,2H), 8.02 (s,1H) |
| 1.7 | 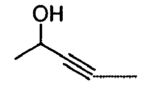 | 1.53 (d,3H), 2.28 (m,2H), 4.12 (t,2H), 4.25 (t,2H), 4.58 (d,2H), 4.74 (m,1H), 6.10 (t,1H), 6.82 (s,2H), 6.86 (d,2H), 7.36 (d,2H) |
| 1.8 | 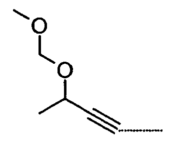 | 1.56 (d,3H), 2.29 (m,2H), 3.40 (s,3H), 4.13 (t,2H), 4.26 (t,2H), 4.58 (d,2H), 4.61-4.70 (m,2H), 5.01 (d,1H), 6.10 (t,1H), 6.82 (s,2H), 6.86 (d,2H), 7.38 (d,2H) |
| 1.9 | 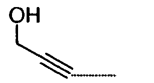 | 1.70 (s,1 H), 2.28 (m,2H), 4.12 (t,2H), 4.35 (t,2H), 4.48 (s,2H), 4.58 (d,2H), 6.11 (t,1 H), 6.84 (s,2H), 6.88 (d,2H), 7.38 (d,2H) |
| 1.10 | 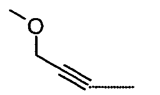 | 2.29 (m,2H), 3.43 (s,3H), 4.13 (t,2H), 4.27 (t,2H), 4.30 (s,2H), 4.58 (d,2H), 6.11 (t,1H), 6.83 (s,2H), 6.88 (d,2H), 7.40 (d,2H |
| 1.11 | 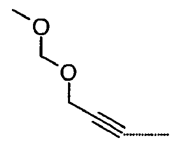 | 2.39 (m,2H), 3.40 (s,3H), 4.13 (t,2H), 4.35 (t,2H), 4.43 (s,2H), 4.58 (d,2H), 4.78 (s,2H), 6.10 (t,1H), 6.82 (s,2H), 6.86 (d,2H), 7.38 (d,2H) |
| 1.12 | 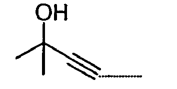 | 1.60 (s,6H), 2.29 (m,2H), 4.12 (t,2H), 4.25 (t,2H), 4.58 (d,2H), 6.10 (t,1H), 6.81 (s,2H), 6.85 (d,2H), 7.36 (d,2H) |
| 1.13 | 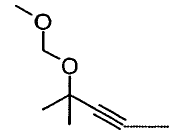 | 1.58 (s,6H), 2.28 (m,2H), 3.42 (s,3H), 4.13 (t,2H), 4.27 (t,2H), 4.58 (d,2H),4.99 (s,2H), 6.10 (t,1H), 6.82 (s,2H), 6.85 (d,2H), 7.36 (d,2H) |
| 1.14 | 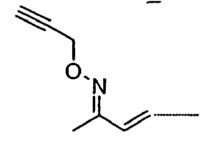 | |
| 1.15 | 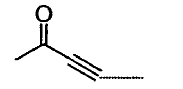 | 2.29 (m,2H), 2.43 (s,3H), 4.13 (t,2H), 4.30 (t,2H), 4.58 (d,2H), 4.74 (m,1H), 6.10 (t,1H), 6.82 (s,2H), 6.92 (d,2H), 7.53 (d,2H) |
| 1.16 | 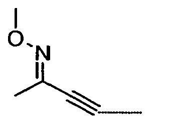 | 2.08+2.10 (s+s,3H), 2.38 (m,2H), 3.97 (s,3H), 4.13 (t,2H), 4.28 (m,2H), 4.56 (d,2H), 6.10 (t,1H), 6.82 (s,2H), 6.88 (m, 2H), 7.47 (m,2H) |
| 1.17 | 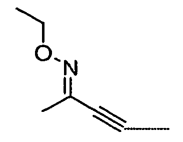 | 1.27-1.39 (m,3H), 2.08+2.10 (s+s,3H), 2.38 (m,2H), 4.09-4.31 (m,6H), 4.57 (d,2H), 6.10 (t,1 H), 6.82 (s,2H), 6.88 (m, 2H), 7.46 (m,2H) |
| 1.18 | 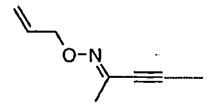 | 2.10 (s,3H), 2.38 (m,2H), 4.14 (m,2H), 4.26 (m,2H), 4.58 (d,2H), 4.65 (m,2H), 5.19-5.40 (m,2H), 5.94-6.13 (m,2H), 6.81 (s,2H), 6.88 (m,2H), 7.43 (m,2H) |
| 1.19 | 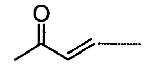 | 2.30 (m,2H), 2.36 (s,3H), 4.16 (t,2H), 4.31 (t,2H), 4.59 (d,2H), 6.11 (t,1 H), 6.61 (d,1 H), 6.82 (s,2H), 6.95 (d,2H), 7.44-7.52 (m, 3H) |
| 1.20 | 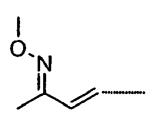 | 2.08 (s,3H), 2.30 (m,2H), 3.93 (s,3H), 4.16 (t,2H), 4.28 (t,2H), 4.59 (d,2H), 6.11 (t,1H), 6.70 (d,1H), 6.75-6.93(m,5H), 7.40 (d,2H) |
| 1.21 | 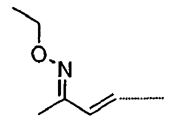 | 1.32 (t,3H), 2.08 (s,3H), 2.30 (m,2H), 4.11-4.22 (m,4H), 4.28 (t,2H), 4.59 (d,2H), 6.10 (t,1H), 6.68-6.92 (m,6H), 7.40 (d,2H) |
| 1.22 | 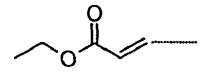 | 1.32 (t,3H), 2.30 (m,2H), 4.15 (t,2H), 4.21-4.33 (m,4H), 4.58 (d,2H), 6.11 (t,1H), 6.30 (d,1H), 6.82 (s,2H), 6.92 (d,2H), 7.48 (d,2H), 7.63 (d,1H) |
| 1.23 | 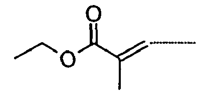 | 1.35 (t,3H), 2.13 (s,3H), 2.30 (m,2H), 4.16 (t,2H), 4.22-4.33 (m,4H), 4.58 (d,2H), 6.10 (t,1H), 6.82 (s,2H), 6.94 (d,2H), 7.39 (d,2H), 7.63 (s,1H) |
| 1.24 | 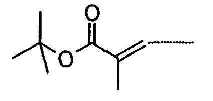 | 1.51 (s,9H), 2.30 (m,2H), 4.15 (t,2H), 4.30 (t,2H), 4.58 (d,2H), 6.10 (t,1H), 6.25 (d,1H), 6.84 (s,2H), 6.92 (d,2H), 7.46 (d,2H), 7.53 (d,1 H) |
| 1.25 | 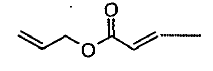 | 2.30 (m,2H), 4.14 (t,2H), 4.30 (t,2H), 4.58 (d,2H), 4.70 (d,2H), 5.28 (d,1H), 5.38 (d,1H), 5.92-6.07 (m,1H), 6.10 (t,1H), 6.34 (d,1H), 6.83 (s,2H), 6.92 (d,2H), 7.49 (d,2H), 7.68 (d,1H) |
| 1.26 | 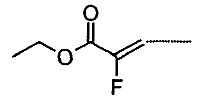 | 1.40 (t,3H), 2.30 (m,2H), 4.16 (t,2H), 4.22-4.32 (m,4H), 4.59 (d,2H),6.11 (t,1H), 6.80-6.93 (m,5H), 7.52 (d,2H) |
| 1.27 | 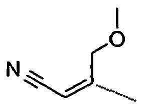 | 2.30 (m,2H), 3.48 (s,2H), 3.78 (s,3H), 4.14 (t,2H), 4.26 (t,2H), 4.58 (d,2H), 6.11 (t,1H), 6.42 (s,1H), 6.82 (s,2H), 6.90 (d,2H), 7.21 (d,2H) |
| 1.28 | 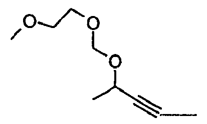 | n_{D}²⁰: 1.5660 |
| 1.29 | 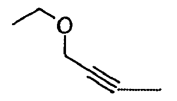 | n_{D}²⁰: 1.5867 |
| 1.30 | 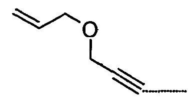 | n_{D}²⁰: 1.5835 |
| 1.31 | 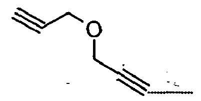 | n_{D}²⁰: 1.5900 |
| 1.32 | 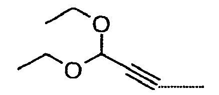 | |
| 1.33 | 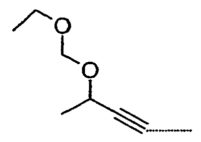 | n_{D}²⁰: 1.5721 |
| 1.34 | 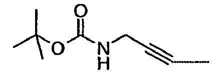 | n_{D}²⁰: 1.5748 |
| 1.35 | 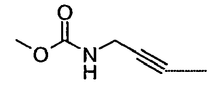 | mp: 78-81 °C |
| 1.36 | 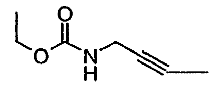 | |
| 1.37 | 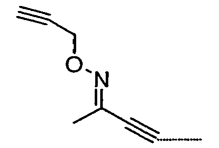 | n_{D}²⁰: 1.6069 |
| 1.38 | 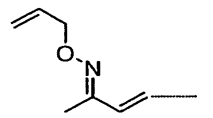 | |
| 1.39 | 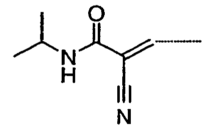 | mp.: 88-93°C |
| 1.40 | 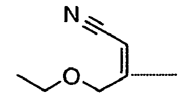 | n_{D}²⁰: 1.5960 |
| 1.41 | 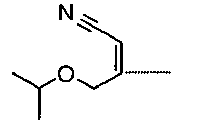 | |
| 1.42 | 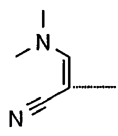 | n_{D}²⁰: 1.6080 |
| 1.43 | 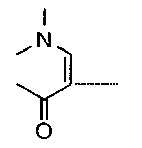 | n_{D}²⁰ 1.5950 |
| 1.44 | 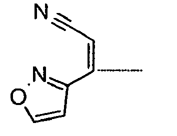 | n_{D}²⁰: 1.6090 |
| 1.45 | 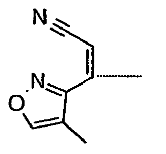 | n_{D}²⁰: 1.6060 |
| 1.46 | 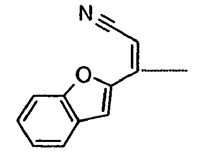 | |
| 1.47 | 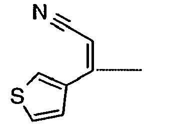 | n_{D}²⁰ 1.6239 |
| 1.48 | 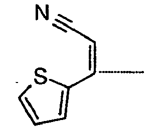 | n_{D}²⁰: 1.6210 |
| 1.49 | 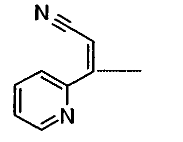 | n_{D}²⁰ 1.6130 |
| 1.50 | 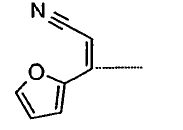 | n_{D}²⁰: 1.5750 |
| 1.51 | 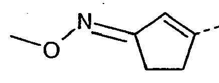 | Mp.: 101-103°C |
| 1.52 | 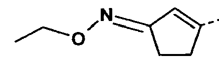 | |
| 1.53 | 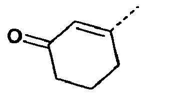 | |
| 1.54 | 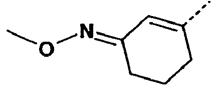 | |

**Table A: Compounds of formulae**

| | |
|---|---|
| 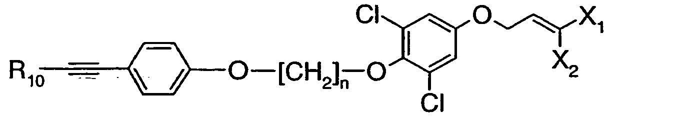 | (Id) |
| 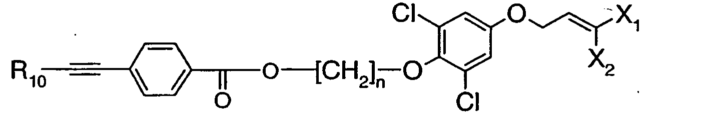 | (Ie) |
| 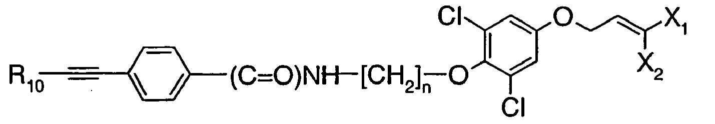 | (If) |
| 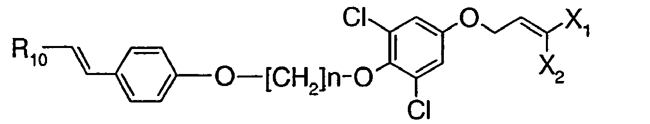 | (Ig) |
| 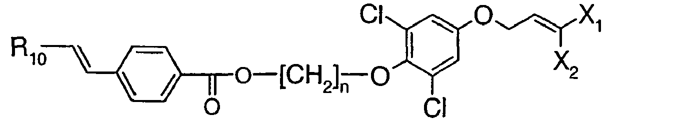 | (lh) |
| 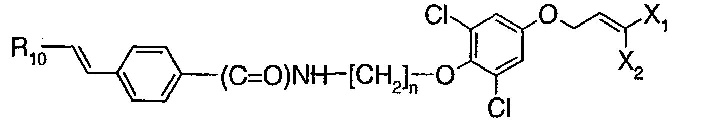 | (li) |
| 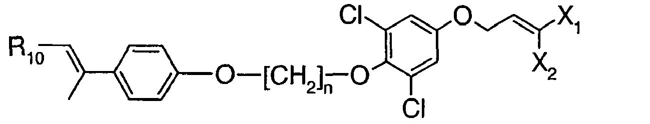 | (lk) |
| 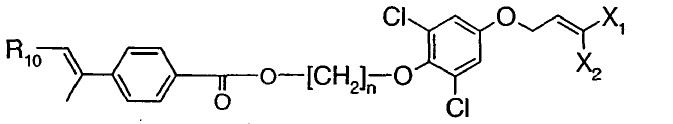 | (lm) |
| 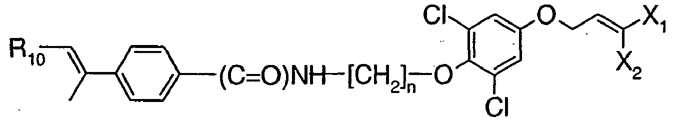 | (ln) |
| 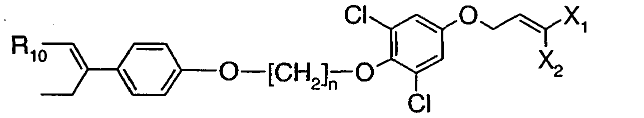 | (lo) |
| 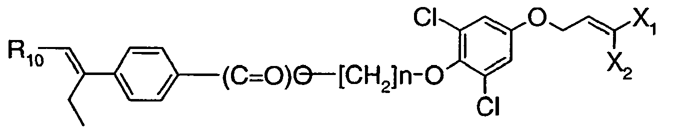 | (lp) |
| 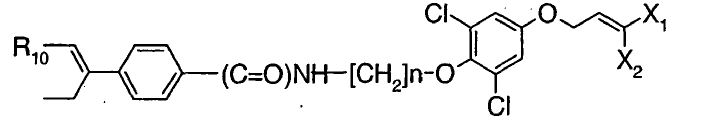 | (lq) |
| 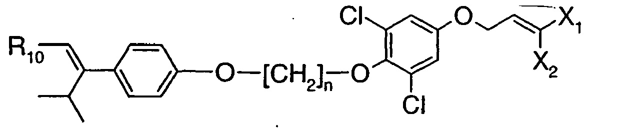 | (lr) |
| 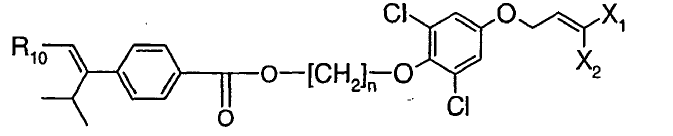 | (ls) |
| 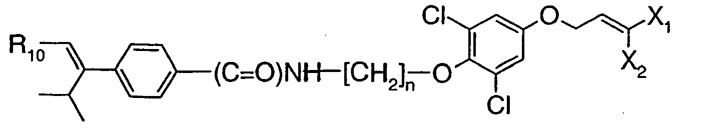 | (lt) |
| 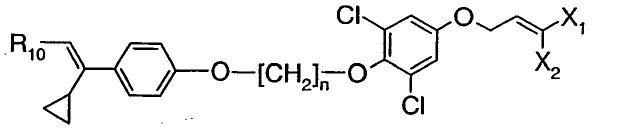 | (lu) |
| 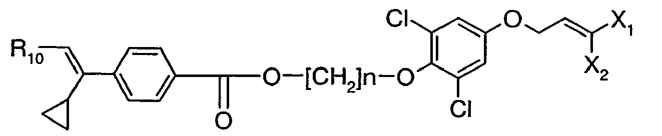 | (lv) |
| 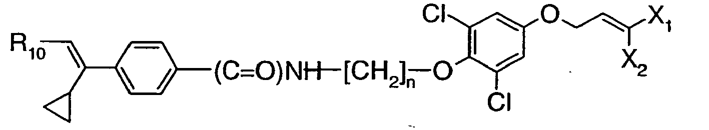 | (lw) |
| 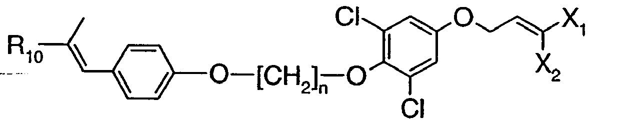 | (lx) |
| 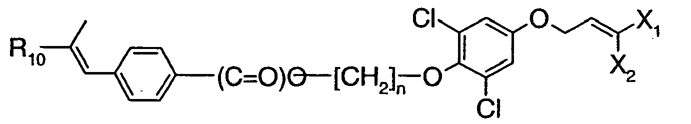 | (ly) |
| 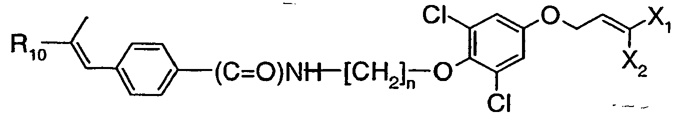 | (lz) |
| 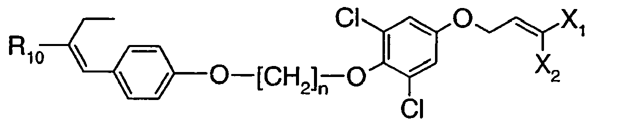 | (laa) |
| 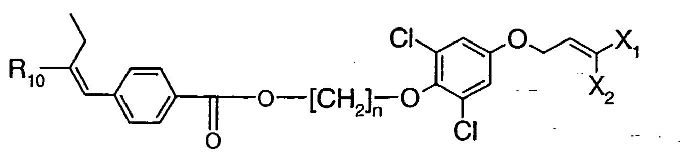 | (lbb) |
| 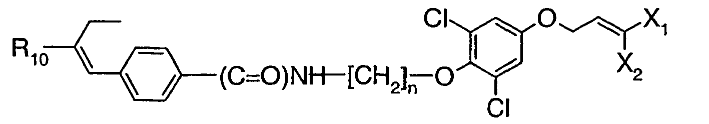 | (Icc) |
| 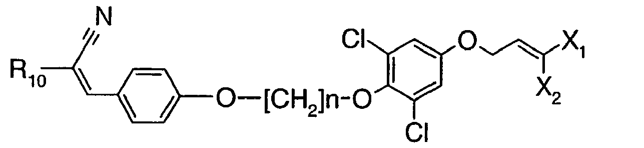 | (ldd) |
| 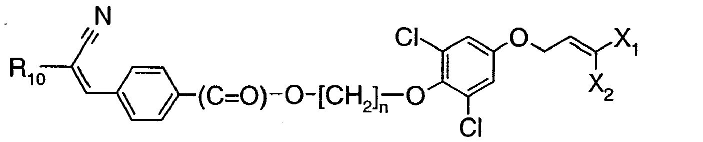 | (lee) |
| 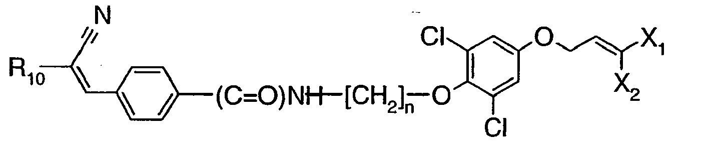 | (Iff) |
| 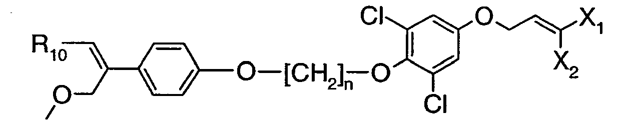 | (Igg) |
| 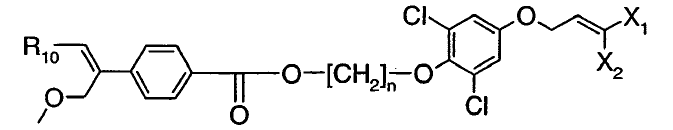 | (Ihh) |
| 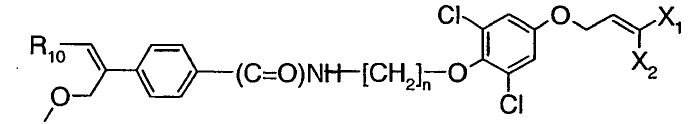 | (lii) |

**Table B:**

| No. | R₁₀ |
|---|---|
| B.1 | CN |
| B.2 | NO₂ |
| B.3 | COOCH₃ |
| B.4 | COOC₂H₅ |
| B.5 | COOn-C₃H₇ |
| B.6 | COOn-C₄H₉ |
| B.7 | COOn-C₅H₁₁ |
| B.8 | COOn-C₆H₁₃ |
| B.9 | COO-iso-C₃H₇ |
| B.10 | COO-iso-C₄H₉ |
| B.11 | COO-iso-C₅H₁₁ |
| B.12 | COO-tert-C₄H₉ |
| B.13 | CH₂OCH₃ |
| B.14 | CH₂OC₂H₅ |
| B.15 | CH₂O-n-C₃H₇ |
| B.16 | CH₂O-n-C₄H₉ |
| B.17 | CH₂O-n-C₅H₁₁ |
| B.18 | CH₂O-n-C₆H₁₃ |
| B.19 | CH₂O-iso-C₃H₇ |
| B.20 | CH₂O-iso-C₄H₉ |
| B.21 | CH₂O-iso-C₅H₁₁ |
| B.22 | CH₂O-tert-C₄H₉ |
| B.23 | CH₂OCH₂C(CH₃)₃ |
| B.24 | CH₂OCH₂(cyclo-propyl) |
| B.25 | CH₂OCF₃ |
| B.26 | CH₂OCH₂CF₃ |
| B.27 | CH₂OCH₂CHF₂ |
| B.28 | CH₂OCH₂CH₂F |
| B.29 | CH₂OCH₂CH=CH₂ |
| B.30 | CH₂OCH₂C≡CH |
| B.31 | CH₂OCH₂C≡CCH₃ |
| B.32 | CH₂OCH₂OCH₃ |
| B.33 | CH₂OCH₂OC₂H₅ |
| B.34 | CH₂OCH₂On-C₃H₇ |
| B.35 | CH₂OCH₂On-C₄H₉ |
| B.36 | CH₂OCH₂On-C₅H₁₁ |
| B.37 | CH₂OCH₂On-C₆H₁₃ |
| B.38 | CH₂OCH₂Oiso-C₃H₇ |
| B.39 | CH₂OCH₂Oiso-C₄H₉ |
| B.40 | CH₂OCH₂Oiso-C₅H₁₁ |
| B.41 | CH₂OCH₂Otert-C₄H₉ |
| B.42 | CH₂OCH₂OCH₂C(CH₃)₃ |
| B.43 | CH₂OCH₂OCH₂(cyclo-propyl) |
| B.44 | CH₂OCH₂OCF₃ |
| B.45 | CH₂OCH₂OCH₂CF₃ |
| B.46 | CH₂OCH₂OCH₂CHF₂ |
| B.47 | CH₂OCH₂OCH₂CH₂F |
| B.48 | CH₂OCH₂OCH₂CH=CH₂ |
| B.49 | CH₂OCH₂OCH₂C≡H |
| B.50 | CH₂OCH₂OCH₂C≡CCH₃ |
| B.51 | CH(CH₃)-OCH₃ |
| B.52 | CH(CH₃)-OC₂H₅ |
| B.53 | CH(CH₃)-On-C₃H₇ |
| B.54 | CH(CH₃)-On-C₄H₉ |
| B.55 | CH(CH₃)-On-C₅H₁₁ |
| B.56 | CH(CH₃)-On-C₆H₁₃ |
| B.57 | CH(CH₃)-Oiso-C₃H₇ |
| B.58 | CH(CH₃)-Oiso-C₄H₉ |
| B.59 | CH(CH₃)-Oiso-C₅H₁₁ |
| B.60 | CH(CH₃)-Otert-C₄H₉ |
| B.61 | CH(CH₃)-OCH₂C(CH₃)₃ |
| B.62 | CH(CH₃)-OCH₂(cyclo-propyl) |
| B.63 | CH(CH₃)-OCF₃ |
| B.64 | CH(CH₃)-OCH₂CF₃ |
| B.65 | CH(CH₃)-OCH₂CHF₂ |
| B.66 | CH(CH₃)-OCH₂CH₂F |
| B.67 | CH(CH₃)-OCH₂CH=CH₂ |
| B.68 | CH(CH₃)-OCH₂C≡CH |
| B.69 | CH(CH₃)-OCH₂C≡CCH₃ |
| B.70 | CH(CH₃)-OCH₂OCH₃ |
| B.71 | CH(CH₃)-OCH₂OC₂H₅ |
| B.72 | CH(CH₃)-OCH₂On-C₃H₇ |
| B.73 | CH(CH₃)-OCH₂On-C₄H₉ |
| B.74 | CH(CH₃)-OCH₂On-C₅H₁₁ |
| B.75 | CH(CH₃)-OCH₂On-C₆H₁₃ |
| B.76 | CH(CH₃)-OCH₂Oiso-C₃H₇ |
| B.77 | CH(CH₃)-OCH₂Oiso-C₄H₉ |
| B.78 | CH(CH₃)-OCH₂Oiso-C₅H₁₁ |
| B.79 | CH(CH₃)-OCH₂Otert-C₄H₉ |
| B.80 | CH(CH₃)-OCH₂OCH₂C(CH₃)₃ |
| B.81 | CH(CH₃)-OCH₂OCH₂(cyclo-propyl) |
| B.82 | CH(CH₃)-OCH₂OCF₃ |
| B.83 | CH(CH₃)-OCH₂OCH₂CF₃ |
| B.84 | CH(CH₃)-OCH₂OCH₂CHF₂ |
| B.85 | CH(CH₃)-OCH₂OCH₂CH₂F |
| B.86 | CH(CH₃)-OCH₂OCH₂CH=CH₂ |
| B.87 | CH(CH₃)-OCH₂OCH₂C≡CH |
| B.88 | CH(CH₃)-OCH₂OCH₂C≡CCH₃ |
| B.89 | C(CH₃)₂-OCH₃ |
| B.90 | C(CH₃)₂-OC₂H₅ |
| B.91 | C(CH₃)₂-On-C₃H₇ |
| B.92 | C(CH₃)₂-On-C₄H₉ |
| B.93 | C(CH₃)₂-On-C₅H₁₁ |
| B.94 | C(CH₃)₂-On-C₆H₁₃ |
| B.95 | C(CH₃)₂-Oiso-C₃H₇ |
| B.96 | C(CH₃)₂-Oiso-C₄H₉ |
| B.97 | C(CH₃)₂-Oiso-C₅H₁₁ |
| B.98 | C(CH₃)₂-Otert-C₄H₉ |
| B.99 | C(CH₃)₂-OCH₂C(CH₃)₃ |
| B.100 | C(CH₃)₂-OCH₂(cyclo-propyl) |
| B.101 | C(CH₃)₂-OCF₃ |
| B.102 | C(CH₃)₂-OCH₂CF₃ |
| B.103 | C(CH₃)₂-OCH₂CHF₂ |
| B.104 | C(CH₃)₂-OCH₂CH₂F |
| B.105 | C(CH₃)₂-OCH₂CH=CH₂ |
| B.106 | C(CH₃)₂-OCH₂C≡CH |
| B.107 | C(CH₃)₂-OCH₂C≡CCH₃ |
| B.108 | C(CH₃)₂-OCH₂OCH₃ |
| B.109 | C(CH₃)₂-OCH₂OC₂H₅ |
| B.110 | C(CH₃)₂-OCH₂On-C₃H₇ |
| B.111 | C(CH₃)₂-OCH₂On-C₄H₉ |
| B.112 | C(CH₃)₂-OCH₂On-C₅H₁₁ |
| B.113 | C(CH₃)₂-OCH₂On-C₆H₁₃ |
| B.114 | C(CH₃)₂-OCH₂Oiso-C₃H₇ |
| B.115 | C(CH₃)₂-OCH₂Oiso-C₄H₉ |
| B.116 | C(CH₃)₂-OCH₂Oiso-C₅H₁₁ |
| B.117 | C(CH₃)₂-OCH₂Otert-C₄H₉ |
| B.118 | C(CH₃)₂-OCH₂OCH₂C(CH₃)₃ |
| B.119 | C(CH₃)₂-OCH₂OCH₂(cyclo-propyl) |
| B.120 | C(CH₃)₂-OCH₂OCF₃ |
| B.121 | C(CH₃)₂-OCH₂OCH₂CF₃ |
| B.122 | C(CH₃)₂-OCH₂OCH₂CHF₂ |
| B.123 | C(CH₃)₂-OCH₂OCH₂CH₂F |
| B.124 | C(CH₃)₂-OCH₂OCH₂CH=CH₂ |
| B.125 | C(CH₃)₂-OCH₂OCH₂C≡CH |
| B.126 | C(CH₃)₂-OCH₂OCH₂C≡CCH₃ |
| B.127 | C(=O)CH₃ |
| B.128 | C(=O)C₂H₅ |
| B.129 | C(=O)-n-C₃H₇ |
| B.130 | C(=O)-n-C₄H₉ |
| B.131 | C(=O)-n-C₅H₁₁ |
| B.132 | C(=O)-n-C₆H₁₃ |
| B.133 | C(=O)-iso-C₃H₇ |
| B.134 | C (=O)-iso-C₄H₉ |
| B.135 | C(=O)-iso-C₅H₁₁ |
| B.136 | C(=O)-tert-C₄H₉ |
| B.137 | C(=O)-cyclo-propyl |
| B.138 | C(=N-OCH₃)CH₃ |
| B.139 | C(=N-OCH₃)C₂H₅ |
| B.140 | C(=N-OCH₃)-n-C₃H₇ |
| B.141 | C(=N-OCH₃)-n-C₄H₉ |
| B.142 | C(=N-OCH₃)-n-C₅H₁₁ |
| B.143 | C(=N-OCH₃)-n-C₆H₁₃ |
| B.144 | C(=N-OCH₃)-iso-C₃H₇ |
| B.145 | C(=N-OCH₃)-iso-C₄H₉ |
| B.146 | C(=N-OCH₃)-iso-C₅H₁₁ |
| B.147 | C(=N-OCH₃)-tert-C₄H₉ |
| B.148 | C(=N-OCH₃)-cyclo-propyl |
| B.149 | C(=N-OCH₂CH₃)CH₃ |
| B.150 | C(=N-OCH₂CH₃)C₂H₅ |
| B.151 | C(=N-OCH₂CH₃)-n-C₃H₇ |
| B.152 | C(=N-OCH₂CH₃)-n-C₄H₉ |
| B.153 | C(=N-OCH₂CH₃)-n-C₅H₁₁ |
| B.154 | C(=N-OCH₂CH₃)-n-C₆H₁₃ |
| B.155 | C(=N-OCH₂CH₃)-iso-C₃H₇ |
| B.156 | C(=N-OCH₂CH₃)-iso-C₄H₉ |
| B.157 | C(=N-OCH₂CH₃)-iso-C₅H₁₁ |
| B.158 | C(=N-OCH₂CH₃)-tert-C₄H₉ |
| B.159 | C(=N-OCH₂CH₃)-cyclo-propyl |
| B.160 | C(=N-OCH₂CH=CH₂)CH₃ |
| B.161 | C(=N-OCH₂CH=CH₂)C₂H₅ |
| B.162 | C(=N-OCH₂CH=CH₂)-n-C₃H₇ |
| B.163 | C(=N-OCH₂CH=CH₂)-n-C₄H₉ |
| B.164 | C(=N-OCH₂CH=CH₂)-n-C₅H₁₁ |
| B.165 | C(=N-OCH₂CH=CH₂)-n-C₆H₁₃ |
| B.166 | C(=N-OCH₂CH=CH₂)-iso-C₃H₇ |
| B.167 | C(=N-OCH₂CH=CH₂)-iso-C₄H₉ |
| B.168 | C(=N-OCH₂CH=CH₂)-iso-C₅H₁₁ |
| B.169 | C(=N-OCH₂CH=CH₂)-tert-C₄H₉ |
| B.170 | C(=N-OCH₂CH=CH₂)-cyclo-propyl |
| B.171 | C(=N-OCH₂C≡CH)CH₃ |
| B.172 | C(=N-OCH₂C≡CH)C₂H₅ |
| B.173 | C(=N-OCH₂C≡CH)n-C₃H₇ |
| B.174 | C(=N-OCH₂C≡CH)n-C₄H₉ |
| B.175 | C(=N-OCH₂C≡CH)n-C₅H₁₁ |
| B.176 | C(=N-OCH₂C≡CH)n-C₆H₁₃ |
| B.177 | C(=N-OCH₂C≡CH)iso-C₃H₇ |
| B.178 | C(=N-OCH₂C≡CH)iso-C₄H₉ |
| B.179 | C(=N-OCH₂C≡CH)iso-C₅H₁₁ |
| B.180 | C(=N-OCH₂C≡CH)tert-C₄H₉ |
| B.181 | C(=N-OCH₂C≡CH)cyclo-propyl |
| B.182 | CONH-CH₃ |
| B.183 | CONH-C₂H₅ |
| B.184 | CONH-n-C₃H₇ |
| B.185 | CONH-n-C₄H₉ |
| B.186 | CONH-n-C₅H₁₁ |
| B.187 | CONH-n-C₆H₁₃ |
| B.188 | CONH-iso-C₃H₇ |
| B.189 | CONH-iso-C₄H₉ |
| B.190 | CONH-iso-C₅H₁₁ |
| B.191 | CONH-tert-C₄H₉ |

Table 2: A compound of the general formula (id) wherein X₁ and X₂ are chlorine and n is 2, and the substituent R₁₀ for each compound corresponds to a line B.1 to B.191 of Table B.
Table 3: A compound of the general formula (Ie) wherein X₁ and X₂ are chlorine and n is 2, and the substituent R₁₀ for each compound corresponds to a line B.1 to B.191 of Table B.
Table 4: A compound of the general formula (If) wherein X₁ and X₂ are chlorine and n is 2, and the substituent R₁₀ for each compound corresponds to a line B.1 to B.191 of Table B.
Table 5: A compound of the general formula (lg) wherein X₁ and X₂ are chlorine and n is 2, and the substituent R₁₀ for each compound corresponds to a line B.1 to B.191 of Table B.
Table 6: A compound of the general formula (lh) wherein X₁ and X₂ are chlorine and n is 2, and the substituent R₁₀ for each compound corresponds to a line B.1 to B.191 of Table B.
Table 7: A compound of the general formula (li) wherein X₁ and X₂ are chlorine and n is 2, and the substituent R₁₀ for each compound corresponds to a line B.1 to B.191 of Table B.
Table 8: A compound of the general formula (lk) wherein X₁ and X₂ are chlorine and n is 2, and the substituent R₁₀ for each compound corresponds to a line B.1 to B.191 of Table B.
Table 9: A compound of the general formula (Im) wherein X₁ and X₂ are chlorine and n is 2, and the substituent R₁₀ for each compound corresponds to a line B.1 to B.191 of Table B.
Table 10: A compound of the general formula (In) wherein X₁ and X₂ are chlorine and n is 2, and the substituent R₁₀ for each compound corresponds to a line B.1 to B.191 of Table B.
Table 11: A compound of the general formula (lo) wherein X₁ and X₂ are chlorine and n is 2, and the substituent R₁₀ for each compound corresponds to a line B.1 to B.191 of Table B.
Table 12: A compound of the general formula (Ip) wherein X₁ and X₂ are chlorine and n is 2, and the substituent R₁₀ for each compound corresponds to a line B.1 to B.191 of Table B.
Table 13: A compound of the general formula (Iq) wherein X₁ and X₂ are chlorine and n is 2, and the substituent R₁₀ for each compound corresponds to a line B.1 to B.191 of Table B.
Table 14: A compound of the general formula (Ir) wherein X₁ and X₂ are chlorine and n is 2, and the substituent R₁₀ for each compound corresponds to a line B.1 to B.191 of Table B.
Table 15: A compound of the general formula (Is) wherein X₁ and X₂ are chlorine and n is 2, and the substituent R₁₀ for each compound corresponds to a line B.1 to B.191 of Table B.
Table 16: A compound of the general formula (It) wherein X₁ and X₂ are chlorine and n is 2, and the substituent R₁₀ for each compound corresponds to a line B.1 to B.191 of Table B.
Table 17: A compound of the general formula (lu) wherein X₁ and X₂ are chlorine and n is 2, and the substituent R₁₀ for each compound corresponds to a line B.1 to B.191 of Table B.
Table 18: A compound of the general formula (Iv) wherein X₁ and X₂ are chlorine and n is 2, and the substituent R₁₀ for each compound corresponds to a line B.1 to B.191 of Table B.
Table 19: A compound of the general formula (Iw) wherein X₁ and X₂ are chlorine and n is 2, and the substituent R₁₀ for each compound corresponds to a line B.1 to B.191 of Table B.
Table 20: A compound of the general formula (Ix) wherein X₁ and X₂ are chlorine and n is 2, and the substituent R₁₀ for each compound corresponds to a line B.1 to B.191 of Table B.
Table 21: A compound of the general formula (Iy) wherein X₁ and X₂ are chlorine and n is 2, and the substituent R₁₀ for each compound corresponds to a line B.1 to B.191 of Table B.
Table 22: A compound of the general formula (Iz) wherein X₁ and X₂ are chlorine and n is 2, and the substituent R₁₀ for each compound corresponds to a line B.1 to B.191 of Table B.
Table 23: A compound of the general formula (Iaa) wherein X₁ and X₂ are chlorine and n is 2, and the substituent R₁₀ for each compound corresponds to a line B.1 to B.191 of Table B.
Table 24: A compound of the general formula (lbb) wherein X₁ and X₂ are chlorine and n is 2, and the substituent R₁₀ for each compound corresponds to a line B.1 to B.191 of Table B.
Table 25: A compound of the general formula (Icc) wherein X₁ and X₂ are chlorine and n is 2, and the substituent R₁₀ for each compound corresponds to a line B.1 to B.191 of Table B.
Table 26: A compound of the general formula (Idd) wherein X₁ and X₂ are chlorine and n is 2, and the substituent R₁₀ for each compound corresponds to a line B.1 to B.191 of Table B.
Table 27: A compound of the general formula (lee) wherein X₁ and X₂ are chlorine and n is 2, and the substituent R₁₀ for each compound corresponds to a line B.1 to B.191 of Table B.
Table 28: A compound of the general formula (Iff) wherein X₁ and X₂ are chlorine and n is 2, and the substituent R₁₀ for each compound corresponds to a line B.1 to B.191 of Table B.
Table 29: A compound of the general formula (Igg) wherein X₁ and X₂ are chlorine and n is 2, and the substituent R₁₀ for each compound corresponds to a line B.1 to B.191 of Table B.
Table 30: A compound of the general formula (Ihh) wherein X₁ and X₂ are chlorine and n is 2, and the substituent R₁₀ for each compound corresponds to a line B.1. to B.191 of Table B.
Table 31: A compound of the general formula (Iii) wherein X₁ and X₂ are chlorine and n is 2, and the substituent R₁₀ for each compound corresponds to a line B.1 to B.191 of Table B.
Table 32: A compound of the general formula (Id) wherein X₁ and X₂ are chlorine and n is 3, and the substituent R₁₀ for each compound corresponds to a line B.1 to B.191 of Table B.
Table 33: A compound of the general formula (Ie) wherein X₁ and X₂ are chlorine and n is 3, and the substituent R₁₀ for each compound corresponds to a line B.1 to B.191 of Table B.
Table 34: A compound of the general formula (If) wherein X₁ and X₂ are chlorine and n is 3, and the substituent R₁₀ for each compound corresponds to a line B.1 to B.191 of Table B.
Table 35: A compound of the general formula (Ig) wherein X₁ and X₂ are chlorine and n is 3, and the substituent R₁₀ for each compound corresponds to a line B.1 to B.191 of Table B.
Table 36: A compound of the general formula (lh) wherein X₁ and X₂ are chlorine and n is 3, and the substituent R₁₀ for each compound corresponds to a line B.1 to B.191 of Table B.
Table 37: A compound of the general formula (Ii) wherein X₁ and X₂ are chlorine and n is 3, and the substituent R₁₀ for each compound corresponds to a line B.1 to B.191 of Table B.
Table 38: A compound of the general formula (lk) wherein X₁ and X₂ are chlorine and n is 3, and the substituent R₁₀ for each compound corresponds to a line B.1 to B.191 of Table B.
Table 39: A compound of the general formula (lm) wherein X₁ and X₂ are chlorine and n is 3, and the substituent R₁₀ for each compound corresponds to a line B.1 to B.191 of Table B.
Table 40: A compound of the general formula (In) wherein X₁ and X₂ are chlorine and n is 3, and the substituent R₁₀ for each compound corresponds to a line B.1 to B.191 of Table B.
Table 41: A compound of the general formula (lo) wherein X₁ and X₂ are chlorine and n is 3, and the substituent R₁₀ for each compound corresponds to a line B.1 to B.191 of Table B.
Table 42: A compound of the general formula (lp) wherein X₁ and X₂ are chlorine and n is 3, and the substituent R₁₀ for each compound corresponds to a line B.1 to B.191 of Table B.
Table 43: A compound of the general formula (Iq) wherein X₁ and X₂ are chlorine and n is 3, and the substituent R₁₀ for each compound corresponds to a line B.1 to B.191 of Table B.
Table 44: A compound of the general formula (Ir) wherein X₁ and X₂ are chlorine and n is 3, and the substituent R₁₀ for each compound corresponds to a line B.1 to B.191 of Table B.
Table 45: A compound of the general formula (Is) wherein X₁ and X₂ are chlorine and n is 3, and the substituent R₁₀ for each compound corresponds to a line B.1 to B.191 of Table B.
Table 46: A compound of the general formula (It) wherein X₁ and X₂ are chlorine and n is 3, and the substituent R₁₀ for each compound corresponds to a line B.1 to B.191 of Table B.
Table 47: A compound of the general formula (Iu) wherein X₁ and X₂ are chlorine and n is 3, and the substituent R₁₀ for each compound corresponds to a line B.1 to B.191 of Table B.
Table 48: A compound of the general formula (Iv) wherein X₁ and X₂ are chlorine and n is 3, and the substituent R₁₀ for each compound corresponds to a line B.1 to B.191 of Table B.
Table 49: A compound of the general formula (lw) wherein X₁ and X₂ are chlorine and n is 3, and the substituent R₁₀ for each compound corresponds to a line B.1 to B.191 of Table B.
Table 50: A compound of the general formula (Ix) wherein X₁ and X₂ are chlorine and n is 3, and the substituent R₁₀ for each compound corresponds to a line B.1 to B.191 of Table B.
Table 51: A compound of the general formula (Iy) wherein X₁ and X₂ are chlorine and n is 3, and the substituent R₁₀ for each compound corresponds to a line B.1 to B.191 of Table B.
Table 52: A compound of the general formula (lz) wherein X₁ and X₂ are chlorine and n is 3, and the substituent R₁₀ for each compound corresponds to a line B.1 to B.191 of Table B.
Table 53: A compound of the general formula (laa) wherein X₁ and X₂ are chlorine and n is 3, and the substituent R₁₀ for each compound corresponds to a line B.1 to B.191 of Table B.
Table 54: A compound of the general formula (Ibb) wherein X₁ and X₂ are chlorine and n is 3, and the substituent R₁₀ for each compound corresponds to a line B.1 to B.191 of Table B.
Table 55: A compound of the general formula (Icc) wherein X₁ and X₂ are chlorine and n is 3, and the substituent R₁₀ for each compound corresponds to a line B.1 to B.191 of Table B.
Table 56: A compound of the general formula (ldd) wherein X₁ and X₂ are chlorine and n is 3, and the substituent R₁₀ for each compound corresponds to a line B.1 to B.191 of Table B.
Table 57: A compound of the general formula (lee) wherein X₁ and X₂ are chlorine and n is 3, and the substituent R₁₀ for each compound corresponds to a line B.1 to B.191 of Table B.
Table 58: A compound of the general formula (lff) wherein X₁ and X₂ are chlorine and n is 3, and the substituent R₁₀ for each compound corresponds to a line B.1 to B.191 of Table B.
Table 59: A compound of the general formula (Igg) wherein X₁ and X₂ are chlorine and n is 3, and the substituent R₁₀ for each compound corresponds to a line B.1 to B.191 of Table B.
Table 60: A compound of the general formula (Ihh) wherein X₁ and X₂ are chlorine and n is 3, and the substituent R₁₀ for each compound corresponds to a line B.1 to B.191 of Table B.
Table 61: A compound of the general formula (iii) wherein X₁ and X₂ are chlorine and n is 3, and the substituent R₁₀ for each compound corresponds to a line B.1 to B.191 of Table B.
Table 62: A compound of the general formula (id) wherein X₁ and X₂ are chlorine and n is 4, and the substituent R₁₀ for each compound corresponds to a line B.1 to B.191 of Table B.
Table 63: A compound of the general formula (le) wherein X₁ and X₂ are chlorine and n is 4, and the substituent R₁₀ for each compound corresponds to a line B.1 to B.191 of Table B.
Table 64: A compound of the general formula (lf) wherein X₁ and X₂ are chlorine and n is 4, and the substituent R₁₀ for each compound corresponds to a line B.1 to B.191 of Table B.
Table 65: A compound of the general formula (Ig) wherein X₁ and X₂ are chlorine and n is 4, and the substituent R₁₀ for each compound corresponds to a line B.1 to B.191 of Table B.
Table 66: A compound of the general formula (Ih) wherein X₁ and X₂ are chlorine and n is 4, and the substituent R₁₀ for each compound corresponds to a line B.1 to B.191 of Table B.
Table 67: A compound of the general formula (ii) wherein X₁ and X₂ are chlorine and n is 4, and the substituent R₁₀ for each compound corresponds to a line B.1 to B.191 of Table B.
Table 68: A compound of the general formula (1k) wherein X₁ and X₂ are chlorine and n is 4, and the substituent R₁₀ for each compound corresponds to a line B.1 to B.191 of Table B.
Table 69: A compound of the general formula (lm) wherein X₁ and X₂ are chlorine and n is 4, and the substituent R₁₀ for each compound corresponds to a line B.1 to B.191 of Table B.
Table 70: A compound of the general formula (In) wherein X₁ and X₂ are chlorine and n is 4, and the substituent R₁₀ for each compound corresponds to a line B.1 to B.191 of Table B.
Table 71: A compound of the general formula (Io) wherein X₁ and X₂ are chlorine and n is 4, and the substituent R₁₀ for each compound corresponds to a line B.1 to B.191 of Table B.
Table 72: A compound of the general formula (Ip) wherein X₁ and X₂ are chlorine and n is 4, and the substituent R₁₀ for each compound corresponds to a line B.1 to B.191 of Table B.
Table 73: A compound of the general formula (Iq) wherein X₁ and X₂ are chlorine and n is 4, and the substituent R₁₀ for each compound corresponds to a line B.1 to B.191 of Table B.
Table 74: A compound of the general formula (Ir) wherein X₁ and X₂ are chlorine and n is 4, and the substituent R₁₀ for each compound corresponds to a line B.1 to B.191 of Table B.
Table 75: A compound of the general formula (Is) wherein X₁ and X₂ are chlorine and n is 4, and the substituent R₁₀ for each compound corresponds to a line B.1 to B.191 of Table B.
Table 76: A compound of the general formula (lt) wherein X₁ and X₂ are chlorine and n is 4, and the substituent R₁₀ for each compound corresponds to a line B.1 to B.191 of Table B.
Table 77: A compound of the general formula (Iu) wherein X₁ and X₂ are chlorine and n is 4, and the substituent R₁₀ for each compound corresponds to a line B.1 to B.191 of Table B.
Table 78: A compound of the general formula (Iv) wherein X₁ and X₂ are chlorine and n is 4, and the substituent R₁₀ for each compound corresponds to a line B.1 to B.191 of Table B.
Table 79: A compound of the general formula (lw) wherein X₁ and X₂ are chlorine and n is 4, and the substituent R₁₀ for each compound corresponds to a line B.1 to B.191 of Table B.
Table 80: A compound of the general formula (lx) wherein X₁ and X₂ are chlorine and n is 4, and the substituent R₁₀ for each compound corresponds to a line B.1 to B.191 of Table B.
Table 81: A compound of the general formula (ly) wherein X₁ and X₂ are chlorine and n is 4, and the substituent R₁₀ for each compound corresponds to a line B.1 to B.191 of Table B.
Table 82: A compound of the general formula (lz) wherein X₁ and X₂ are chlorine and n is 4, and the substituent R₁₀ for each compound corresponds to a line B.1 to B.191 of Table B.
Table 83: A compound of the general formula (laa) wherein X₁ and X₂ are chlorine and n is 4, and the substituent R₁₀ for each compound corresponds to a line B.1 to B.191 of Table B.
Table 84: A compound of the general formula (Ibb) wherein X₁ and X₂ are chlorine and n is 4, and the substituent R₁₀ for each compound corresponds to a line B.1 to B.191 of Table B.
Table 85: A compound of the general formula (lcc) wherein X₁ and X₂ are chlorine and n is 4, and the substituent R₁₀ for each compound corresponds to a line B.1 to B.191 of Table B.
Table 86: A compound of the general formula (ldd) wherein X₁ and X₂ are chlorine and n is 4, and the substituent R₁₀ for each compound corresponds to a line B.1 to B.191 of Table B.
Table 87: A compound of the general formula (lee) wherein X₁ and X₂ are chlorine and n is 4, and the substituent R₁₀ for each compound corresponds to a line B.1 to B.191 of Table B.
Table 88: A compound of the general formula (Iff) wherein X₁ and X₂ are chlorine and n is 4, and the substituent R₁₀ for each compound corresponds to a line B.1 to B.191 of Table B.
Table 89: A compound of the general formula (lgg) wherein X₁ and X₂ are chlorine and n is 4, and the substituent R₁₀ for each compound corresponds to a line B.1 to B.191 of Table B.
Table 90: A compound of the general formula (lhh) wherein X₁ and X₂ are chlorine and n is 4, and the substituent R₁₀ for each compound corresponds to a line B.1 to B.191 of Table B.
Table 91: A compound of the general formula (lii) wherein X₁ and X₂ are chlorine and n is 4, and the substituent R₁₀ for each compound corresponds to a line B.1 to B.191 of Table B.

### Formulation Examples (% = percent by weight)

| Example F1: Emulsifiable concentrates | a) | b) | c) |
|---|---|---|---|
| active ingredient | 25 % | 40 % | 50 % |
| calcium dodecylbenzenesulfonate | 5 % | 8 % | 6 % |
| castor oil polyethylene glycol ether (36 mol EO) | 5 % | - | - |
| tributylphenol polyethylene glycol ether (30 mol EO) | - | 12 % | 4 % |
| cyclohexanone | - | 15 % | 20 % |
| xylene mixture | 65 % | 25 % | 20 % |

Mixing finely ground active ingredient and additives gives an emulsifiable concentrate which yields emulsions of the desired concentration on dilution with water.

| Example F2: Solutions | a) | b) | c) | d) |
|---|---|---|---|---|
| active ingredient | 80% | 10% | 5% | 95% |
| ethylene glycol monomethyl ether | 20 % | - | - | - |
| polyethylene glycol (MW 400) | - | 70 % | - | - |
| N-methylpyrrolid-2-one | - | 20 % | - | - |
| - epoxidised coconut oil | - | - | 1 % | 5 % |
| benzine (boiling range: 160-190°) | - | - | 94 % | - |

Mixing finely ground active ingredient and additives gives a solution suitable for use in the form of microdrops.

| Example F3: Granules | a) | b) - | c) | d) |
|---|---|---|---|---|
| active ingredient | 5 % | 10 % | 8 % | 21 % |
| kaolin | 94 % | - | 79 % | 54 % |
| highly dispersed silicic acid | 1 % | - | 13 % | 7 % |
| attapulgite | - | 90 % | - | 18 % |

The active ingredient is dissolved in dichloromethane, the solution is sprayed onto the carrier mixture and the solvent is evaporated off *in vacuo.*

### Biological Examples

### Example B1: Action against Heliothis virescens caterpillars

Young soybean plants are sprayed with an aqueous emulsion spray mixture comprising 400 ppm of active ingredient. After the spray-coating has dried, the soybean plants are populated with 10 caterpillars of Heliothis virescens in the first stage and placed in a plastics container. Evaluation is made 6 days later. The percentage reduction in population and the percentage reduction in feeding damage (% activity) are determined by comparing the number of dead caterpillars and the feeding damage on the treated plants with that on untreated plants.

The compounds of the Tables exhibit good activity against Heliothis virescens in this test. In particular, the compounds 1.1 to 1.13 and 1.15 to 1.27 are more than 80 % effective.

### Example B2: Action against Plutella xylostella caterpillars

Young cabbage plants are sprayed with an aqueous emulsion spray mixture comprising 400 ppm of active ingredient. After the spray-coating has dried, the cabbage plants are populated with 10 caterpillars of Plutella xylostella in the third stage and placed in a plastics container. Evaluation is made 3 days later. The percentage reduction in population and the percentage reduction in feeding damage (% activity) are determined by comparing the number of dead caterpillars and the feeding damage on the treated plants with that on untreated plants.

The compounds of the Tables exhibit good activity against Plutella xylostella in this test. In particular, the compounds 1.1 to 1.13 and 1.15 to 1.27 are more than 80 % effective.

### Example B3: Action against Spodoptera littoralis

Young soybean plants are sprayed with an aqueous emulsion spray mixture comprising 400 ppm of active ingredient and, after the spray-coating has dried, the plants are populated with 10 caterpillars of Spodoptera littoralis in the first stage and then placed in a plastics container. 3 days later, the percentage reduction in population and the percentage reduction in feeding damage (% activity) are determined by comparing the number of dead caterpillars and the feeding damage on the treated plants with that on untreated plants.

The compounds of the Tables exhibit good activity against Spodoptera littoralis in this test. In particular, the compounds 1.1 to 1.13 and 1.15 to 1.27 are more than 80 % effective.

## Claims

1. A compound of formula 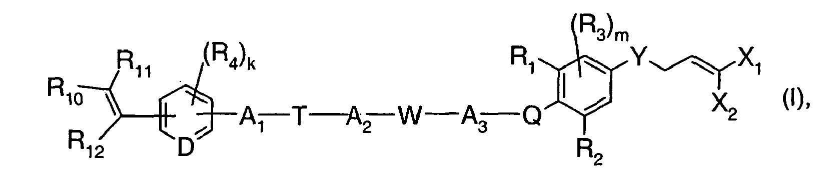 wherein
A₁ and A₂ are each independently of the other a bond or a C₁-C₆alkylene bridge which is unsubstituted or substituted by from one to six identical or different substituents selected from halogen and C₃-C₈cycloalkyl;
A₃ is a C₁-C₆alkylene bridge which is unsubstituted or substituted by from one to six identical or different substituents selected from halogen and C₃-C₈cycloalkyl;
Y is O, NR₇, S, SO or SO₂;
X₁ and X₂ are each independently of the other fluorine, chlorine or bromine;
R₁, R₂ and R₃ are each independently of the others H, halogen, OH, SH, CN, nitro, C₁-C₆alkyl, C₁-C₆haloalkyl, C₁-C₆alkylcarbonyl, C₂-C₆alkenyl, C₂-C₆haloalkenyl, C₂-C₆alkynyl, C₁-C₆alkoxy, C₁-C₆haloalkoxy, C₃-C₆alkenyloxy, C₃-C₆haloalkenyloxy, C₃-C₆alkynyloxy, -(S=O)-C₁=C₆alkyl, -(SO)₂-C₁-C₆alkyl or C₁-C₆alkoxycarbonyl; the substituents R₃ being independent of one another when m is 2;
Q is O, NR₅, S, SO or SO₂;
W is O, NR₅, S, SO, SO₂ -C(=O)-O-, -O-C(=O)-, -C(=O)-NR₅- or -NR₅-C(=O)-;
T is a bond, O, NR₅, S, SO, SO₂, -C(=O)-O-, -O-C(=O)-, -C(=O)-NR₅- or -NR₅-C(=O)-;
D is CH or N;
R₄ is H, halogen, OH, SH, CN, nitro, C₁-C₆alkyl, C₁-C₆haloalkyl, C₁-C₆alkylcarbonyl, C₂-C₆alkenyl, C₂-C₆haloalkenyl, C₂-C₆alkynyl, C₁-C₆alkoxy, C₁-C₆haloalkoxy, C₃-C₆-alkenyloxy, C₃-C₆haloalkenyloxy, C₃-C₆alkynyloxy, -( S=O)-C₁-C₆alkyl, -(SO)₂-C₁-C₆alkyl, C₁-C₆alkoxycarbonyl or N(R₆)₂ wherein the two substituents R₆ are independent of one another; the substituents R₄ being independent of one another when k is greater than 1;
R₅, R₆ and R₇ are each independently of the others H, C₁-C₆alkyl, C₁-C₃haloalkyl, C₁-C₃haloalkylcarbonyl, C₁-C₆alkoxyalkyl, C₁-C₆alkylcarbonyl, C₁-C₆alkoxycarbonyl, C₃-C₈-cycloalkyl, C₃-C₈cycloalkyl-C₁-C₆alkyl, C₃-C₈cycloalkylcarbonyl;
k is 1, 2 or 3 when D is nitrogen; or is 1, 2, 3 or 4 when D is CH;
m is 1 or 2;
R₁₀ is any radical which comprises from one to three hetero atoms selected from O, N and S; and which may be connected to R₁₂ via a C₁-C₆alkylene bridge;
R₁₁ is H, C₁-C₁₂alkyl, halogen or any radical which comprises from one to three hetero atoms selected from O, N and S; or R₁₁ together with R₁₂ is a bond;
or R₁₀ and R₁₁, together with the carbon atom to which they are bonded, are a five- to seven-membered ring which optionally contains from one to three hetero atoms selected from O, N and S and which is unsubstituted or substituted by from one to three identical or different substituents selected from halogen, OH, =O, SH, =S, =N-OH, =N-O-C₁-C₆alkyl, CN, nitro, C₁-C₆alkyl, C₁-C₆haloalkyl, C₁-C₆alkylcarbonyl, C₂-C₆alkenyl, C₂-C₆haloalkenyl, C₂-C₆alkynyl, C₁-C₆alkoxy and C₁-C₆haloalkoxy;
R₁₂ is H, C₁-C₆alkyl, halo-C₁-C₆alkyl, C₁-C₆alkoxy-C₁-C₆alkyl, C₃-C₆cycloalkyl, phenoxy-C₁-C₆alkyl, CN, -C(=O)C₁-C₁₂alkyl, unsubstituted heterocyclyl, heterocyclyl which is substituted by one to three substituents selected form the group consisting of OH, =O, SH, =S, halogen, CN, nitro, C₁-C₆alkyl, C₁-C₆haloalkyl, C₁-C₆alkylcarbonyl, C₂-C₆alkenyl, C₂-C₆haloalkenyl, C₁-C₆alkoxy and C₁-C₆haloalkoxy; or R₁₂ together with R₁₁ a bond; or is a C₂-C₆alkylene bridge which is connected to R₁₀;
and, where applicable, their possible E/Z isomers, E/Z isomeric mixtures and/or tautomers, in each case in free form or in salt form.

2. A compound of formula (I) according to claim 1 in free form.

3. A compound of formula (I) according claim 2, wherein X₁ and X₂ are chlorine or bromine.

4. A compound of formula (I) according to claim 3, wherein D is CH.

5. A compound of formula (I) according claim 4, wherein A₃ is propylene.

6. A compound of formula (I) according to claim 1, wherein R₁₁ and R₁₂ together are a bond.

7. A pesticidal composition which comprises as active ingredient at least one compound of formula (I) according to claim 1 in free form or in agrochemically acceptable salt form, and at least one adjuvant.

8. A method of controlling pests, which comprises applying a pesticidal composition as described in claim 7 to the pests or to the locus thereof.

## Patentansprüche

1. Verbindung der Formel 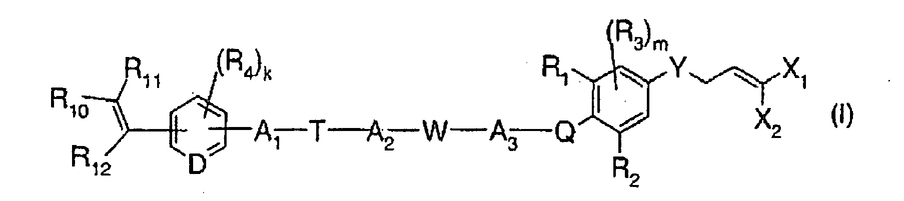 worin
A₁ und A₂ jeweils unabhängig voneinander eine Bindung oder eine C₁-C₆-Alkylenbrücke darstellen, die unsubstituiert oder mit einem bis sechs gleichen oder verschiedenen Substituenten, ausgewählt aus Halogen und C₃-C₈-Cycloalkyl, substituiert ist;
A₃ eine C₁-C₆-Alkylenbrücke darstellt, die unsubstituiert oder mit einem bis sechs gleichen oder verschiedenen Substituenten, ausgewählt aus Halogen und C₃-C₈-Cycloalkyl, substituiert ist;
Y O, NR₇, S, SO oder SO₂ darstellt;
X₁ und X₂ jeweils unabhängig voneinander Fluor, Chlor oder Brom darstellen;
R₁, R₂ und R₃ jeweils unabhängig voneinander H, Halogen, OH, SH, CN, Nitro, C₁-C₆-Alkyl, C₁-C₆-Halogenalkyl, C₁-C₆-Alkylcarbonyl, C₂-C₆-Alkenyl, C₂-C₆-Halogenalkenyl , C₂-C₆-Alkinyl, C₁-C₆-Alkoxy, C₁-C₆-Halogenalkoxy, C₃-C₆-Alkenyloxy, C₃-C₆-Halogenalkenyloxy, C₃-C₆-Alkinyloxy, - (S=O) -C₁-C₆-Alkyl , - (SO)₂-C₁-C₆-Alkyl oder C₁-C₆-Alkoxycarbonyl darstellen; wobei die Substituenten R₃ unabhängig voneinander sind, wenn m 2 ist;
Q O, NR₅, S, SO oder SO₂ darstellt;
W O, NR₅, S, SO, SO₂, -C (=O)-O-, -O-C (=O)-, -C (=O)-NR₅-oder -NR₅-C(=O)- darstellt;
T eine Bindung, O, NR₅, S, SO, SO₂, -C(=O)-O-, -O-C(=O)-, -C(=O)-NR₅- oder -NR₅-C(=O)- darstellt;
D CH oder N darstellt;
R₄ H, Halogen, OH, SH, CN, Nitro, C₁-C₆-Alkyl, C₁-C₆-Halogenalkyl, C₁-C₆-Alkylcarbonyl, C₂-C₆-Alkenyl, C₂-C₆-Halogenalkenyl, C₂-C₆-Alkinyl, C₁-C₆-Alkoxy, C₁-C₆-Halogenalkoxy, C₃-C₆-Alkenyloxy, C₃-C₆-Halogenalkenyloxy, C₃-C₆-Alkinyloxy, - (S=O) -C₁-C₆-Alkyl, - (SO)₂-C₁-C₆-Alkyl, C₁-C₆-Alkoxycarbonyl oder N(R₆)₂ darstellt, worin die zwei Substituenten R₆ unabhängig voneinander sind; wobei die Substituenten R₄ unabhängig voneinander sind, wenn k größer als 1 ist;
R₅, R₆ und R₇ jeweils unabhängig voneinander H, C₁-C₆-Alkyl, C₁-C₃-Halogenalkyl, C₁-C₃-Halogenalkylcarbonyl, C₁-C₆-Alkoxyalkyl , C₁-C₆-Alkylcarbonyl, C₁-C₆-Alkoxycarbonyl, C₃-C₈-Cycloalkyl, C₃-C₈-Cycloalkyl-C₁-C₆-alkyl, C₃-C₈-Cycloalkylcarbonyl darstellen;
k 1, 2 oder 3 ist, wenn D Stickstoff darstellt; oder 1, 2, 3 oder 4 ist, wenn D CH darstellt;
m 1 oder 2 ist;
R₁₀ einen beliebigen Rest darstellt, der ein bis drei Heteroatome, ausgewählt aus O, N und S, umfasst; und der an R₁₂ über eine C₁-C₆-Alkylenbrücke verbunden sein kann;
R₁₁ H, C₁-C₁₂-Alkyl, Halogen oder einen beliebigen Rest darstellt, der ein bis drei Heteroatome, ausgewählt aus O, N und S, umfasst; oder R₁₁ zusammen mit R₁₂ eine Bindung darstellt;
oder R₁₀ und R₁₁ zusammen mit dem Kohlenstoffatom, an das sie gebunden sind, einen fünf- bis siebengliedrigen Ring darstellen, der gegebenenfalls ein bis drei Heteroatome, ausgewählt aus O, N und S, enthält, und unsubstituiert oder mit einem bis drei gleichen oder verschiedenen Substituenten, ausgewählt aus Halogen, OH, =O, SH, =S, =N-OH, =N-O-C₁-C₆-Alkyl, CN, Nitro, C₁-C₆-Alkyl, C₁-C₆-Halogenalkyl, C₁-C₆-Alkylcarbonyl, C₂-C₆-Alkenyl, C₂-C₆-Halogenalkenyl, C₂-C₆-Alkinyl , C₁-C₆-Alkoxy und C₁-C₆-Halogenalkoxy, substituiert ist;
R₁₂ H, C₁-C₆ Alkyl, Halogen-C₁-C₆-alkyl, C₁-C₆-Alkoxy-C₁-C₆-alkyl, C₃-C₆-Cycloalkyl, Phenoxy-C₁-C₆-alkyl, CN, -C(=O) C₁-C₁₂-Alkyl, unsubstituiertes Heterocyclyl, Heterocyclyl, das mit einem bis drei Substituenten, ausgewählt aus der Gruppe, bestehend aus OH, =O, SH, =S, Halogen, CN, Nitro, C₁-C₆-Alkyl, C₁-C₆-Halogenalkyl, C₁-C₆-Alkylcarbonyl, C₂-C₆-Alkenyl, C₂-C₆-Halogenalkenyl, C₁C₆-Alkoxy und C₁-C₆-Halogenalkoxy, substituiert ist, darstellt; oder R₁₂ zusammen mit R₁₁ eine Bindung darstellt; oder eine C₂-C₆-Alkylenbrücke darstellt, die an R₁₀ gebunden ist;
und, sofern zutreffend, deren mögliche E/Z-Isomeren, E/Z-Isomerengemische und/oder Tautomere, jeweils in freier Form oder in Salzform.

2. Verbindung der Formel (I) nach Anspruch 1 in freier Form.

3. Verbindung der Formel (I) nach Anspruch 2, worin X₁ und X₂ Chlor oder Brom darstellen.

4. Verbindung der Formel (I) nach Anspruch 3, worin D CH darstellt.

5. Verbindung der Formel (I) nach Anspruch 4, worin A₃ Propylen darstellt.

6. Verbindung der Formel (I) nach Anspruch 1, worin R₁₁ und R₁₂ zusammen eine Bindung darstellen.

7. Pestizide Zusammensetzung, die als Wirkstoff mindestens eine Verbindung der Formel (I) nach Anspruch 1 in freier Form oder in agrochemisch verträglicher Salzform und mindestens einen Hilfsstoff umfasst.

8. Verfahren zum Bekämpfen von Schädlingen, das Applizieren einer pestiziden Zusammensetzung, wie in Anspruch 7 beschrieben, auf die Schädlinge oder deren Standort umfasst.

## Revendications

1. Composé de formule 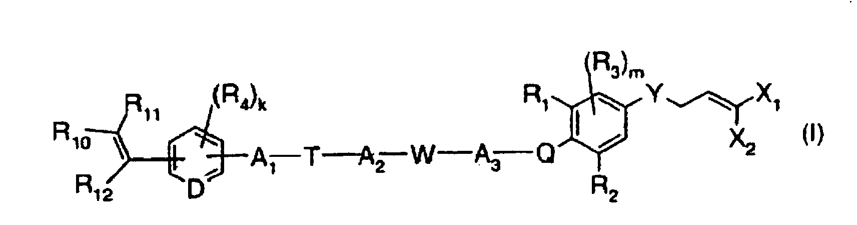 dans laquelle
A₁ et A₂ sont chacun indépendamment de l'autre une liaison ou un pont alkylène en C₁ à C₆ qui est non substitué ou substitué par un à six substituants identiques ou différents choisis parmi un atome d'halogène et un groupe cycloalkyle en C₃ à C₈ ;
A₃ est un pont alkylène en C₁ à C₆ qui est non substitué ou substitué par un à six substituants identiques ou différents choisis parmi un atome d'halogène et un groupe cycloalkyle en C₃ à C₈ ;
Y est O, NR₇, S, SO ou SO₂ ;
X₁ et X₂ sont chacun indépendamment de l'autre un atome de fluor, de chlore ou de brome ;
R₁, R₂ et R₃ sont chacun indépendamment des autres H, un atome d'halogène, un groupe OH, SH, CN, nitro, alkyle en C₁ à C₆, halogénoalkyle en C₁ à C₆, alkylcarbonyle en C₁ à C₆, alcényle en C₂ à C₆, halogénoalcényle en C₂ à C₆, alcynyle en C₂ à C₆, alcoxy en C₁ à C₆, halogénoalcoxy en C₁ à C₆, alcényloxy en C₃ à C₆, halogénoalcényloxy en C₃ à C₆, alcynyloxy en C₃ à C₆, -(S=O)- alkyle en C₁ à C₆, -(SO)₂-alkyle en C₁ à C₆ ou alcoxycarbonyle en C₁ à C₆ ; les substituants R₃ étant indépendants les uns des autres lorsque m vaut 2 ;
Q est O, NR₅, S, SO ou SO₂ ;
W est O, NR₅, S, SO ou SO₂, -C(=O)-O-, -O-C(=O)-, -C(=O)-NR₅- ou -NR₅-C(=O)- ;
T est une liaison, O, NR₅, S, SO, SO₂, -C(=O)-O-, -O-C(=O)-, -C(=O)-NR₅- ou -NR₅-C(=O)- ;
D est CH ou N ;
R₄ est H, un atome d'halogène, un groupe OH, SH, CN, nitro, alkyle en C₁ à C₆, halogénoalkyle en C₁ à C₆, alkylcarbonyle en C₁ à C₆, alcényle en C₂ à C₆, halogénoalcényle en C₂ à C₆, alcynyle en C₂ à C₆, alcoxy en C₁ à C₆, halogénoalcoxy en C₁ à C₆, alcényloxy en C₃ à C₆, halogénoalcényloxy en C₃ à C₆, alcynyloxy en C₃ à C₆, -(S=O)-alkyle en C₁ à C₆, -(SO)₂-alkyle en C₁ à C₆ ou alcoxycarbonyle en C₁ à C₆ ou N(R₆)₂ où les deux substituants R₆ sont indépendants l'un de l'autre ; les substituants R₄ étant indépendants les uns des autres lorsque k est supérieur à 1 ;
R₅, R₆ et R₇ sont chacun indépendamment des autres H, un groupe alkyle en C₁ à C₆, halogénoalkyle en C₁ à C₃, halogénoalkylcarbonyle en C₁ à C₃, alcoxyalkyle en C₁ à C₆, alkylcarbonyle en C₁ à C₆, alcoxycarbonyle en C₁ à C₆, cycloalkyle en C₃ à C₈, cycloalkyle en C₃ à C₈, alkyle en C₁ à C₆, cycloalkylcarbonyle en C₃ à C₈ ;
k vaut 1, 2 ou 3 lorsque D est un atome d'azote ; ou vaut 1, 2, 3 ou 4 lorsque D est CH;
m vaut 1 ou 2 ;
R₁₀ est tout radical qui comprend un à trois hétéroatomes choisis parmi O, N et S ; et qui peut être relié à R₁₂ via un pont alkylène en C₁ à C₆ ;
R₁₁ est H, un groupe alkyle en C₁ à C₁₂, un atome d'halogène ou tout radical qui comprend d'un à trois hétéro atomes choisis parmi O, N et S ; ou R₁₁ conjointement avec R₁₂ est une liaison ;
ou R₁₀ et R₁₁, conjointement avec l'atome de carbone auquel ils sont liés, sont un cycle à cinq à sept chaînons qui contient facultativement d'un à trois hétéro atomes choisis parmi O, N et S et qui est non substitué ou substitué par un à trois substituants identiques ou différents choisis parmi un atome d'halogène, OH, =O, SH, =S, =N-OH, =N-O-alkyle en C₁ à C₆, CN, nitro, alkyle en C₁ à C₆, halogénoalkyle en C₁ à C₆, alkylcarbonyle en C₁ à C₆, alcényle en C₂ à C₆, halogénoalcényle en C₂ à C₆, alcynyle en C₂ à C₆, alcoxy en C₁ à C₆, et halogénoalcoxy enC₁ à C₆;
R₁₂ est H, un groupe alkyle en C₁ à C₆, halogénoalkyle en C₁ à C₆, alcoxy en C₁ à C₆-alkyle en C₁ à C₆, cycloalkyle en C₃ à C₆, phénoxyalkyle en C₁ à C₆, CN, -C(=O)-alkyle en C₁ à C₁₂, hétérocyclyle non substitué, hétérocyclyle qui est substitué par un à trois substituants choisis dans le groupe consistant en OH, =O, SH, =S, un atome d'halogène, un groupe CN, nitro, alkyle en C₁ à C₆, halogénoalkyle en C₁ à C₆, alkylcarbonyle en C₁à C₆, alcényle en C₂ à C₆, halogénoalcényle en C₂ à C₆, alcoxy en C₁ à C₆, et halogénoalcoxy en C₁ à C₆ ; ou R₁₂ conjointement avec R₁₁ est une liaison ; ou est un pont alkylène en C₂ à C₆ qui est relié à R₁₀ ;
et, lorsque cela est applicable, leurs isomères E/Z, mélanges isomères E/Z et/ou tautomères possibles dans chaque cas sous forme libre ou sous forme de sel.

2. Composé de formule (1) selon la revendication 1, sous forme libre.

3. Composé de formule (1) selon la revendication 2, dans lequel X₁ et X₂ sont un atome de chlore ou de brome.

4. Composé de formule (1) selon la revendication 3, dans lequel D est CH.

5. Composé de formule (1) selon la revendication 4, dans lequel A₃ est le propylène.

6. Composé de formule (1) selon la revendication 1, dans lequel R₁₁ et R₁₂ sont ensemble une liaison.

7. Composition pesticide qui comprend comme ingrédient actif au moins un composé de formule (1) selon la revendication 1 sous forme libre ou sous forme de sel agrochimiquement acceptable, et au moins un adjuvant.

8. Procédé de lutte contre les organismes nuisibles, qui comprend l'application d'une composition pesticide telle que décrite dans la revendication 7 aux organismes nuisibles ou au lieu de ceux-ci.
